# EUROPEAN PATENT SPECIFICATION

(11) **EP 3 082 870 B1**
(45) Date of publication and mention of the grant of the patent: **23.10.2019**
(21) Application number: 14819058.0
(22) Date of filing: 19.12.2014
(51) Int. Cl.: A61K 47/68

(54) **CONJUGATES FOR TREATING INFLAMMATORY DISEASE AND IDENTIFICATION OF PATIENTS LIKELY TO BENEFIT FROM SUCH TREATMENT**
KONJUGATE ZUR BEHANDLUNG ENTZÜNDLICHER ERKRANKUNGEN UND IDENTIFIZIERUNG VON PATIENTEN, DIE WAHRSCHEINLICH VON EINER DERARTIGEN BEHANDLUNG PROFITIEREN
CONJUGUÉS POUR LE TRAITEMENT DE MALADIE INFLAMMATOIRE ET IDENTIFICATION DE PATIENTS SUSCEPTIBLES DE BÉNÉFICIER D'UN TEL TRAITEMENT

(30) Priority: 20.12.2013 US 201361919404 P
(43) Date of publication of application: 26.10.2016
(73) Proprietor: The University of Bristol, Bristol BS8 1TH (GB); The Government of the United States of America, as represented by the Secretary of the Department of Health and Human Services, Bethesda, Maryland 20892 (US)
(72) Inventor: LEE, Richard, Bristol, BS8 1TD (GB); ANDREW, Dick, Bristol, BS8 1TD (GB); BOWERS, Lauren Schewitz, Bristol, BS8 1TD (GB); NUSSENBLATT, Robert, deceased (US); GERY, Igal, Bethesda, Maryland 20892 (US); WEI, Lai, Bethesda, Maryland 20892 (US)
(74) Representative: Mewburn Ellis LLP
(86) International application number: PCT/GB2014/053804
(87) International publication number: WO 2015/092435

(56) References cited:
- EP-A1- 2 116 263
- WO-A1-2013/168876
- WO-A2-03/006619
- WO-A2-2010/065962
- WO-A2-2013/102042
- US-A1- 2002 094 542
- STEWARD-THARP S M ET AL: "New insights into T cell biology and T cell-directed therapy for autoimmunity, inflammation, and immunosuppression", ANNALS OF THE NEW YORK ACADEMY OF SCIENCES - THE YEAR IN IMMUNOLOGY 2 20100110 BLACKWELL PUBLISHING INC. USA, vol. 1183, 10 January 2010 (2010-01-10), pages 123-148, XP002742413, ISSN: 0077-8923
- SUSAN E. LACY ET AL: "Americas Antibody Congress", MABS, vol. 1, no. 6, 1 November 2009 (2009-11-01), pages 523-530, XP055199332, US ISSN: 1942-0862, DOI: 10.4161/mabs.1.6.10221
- KUYPERS D R J ET AL: "The use of an anti-CD25 monoclonal antibody and mycophenolate mofetil enables the use of a low-dose tacrolimus and early withdrawal of steroids in renal transplant recipients", CLINICAL TRANSPLANTATION 200306 GB, vol. 17, no. 3, June 2003 (2003-06), pages 234-241, XP002742414, ISSN: 0902-0063
- HEDRICK M N ET AL: "CCR6 as a possible therapeutic target in psoriasis", EXPERT OPINION ON THERAPEUTIC TARGETS, INFORMA HEALTHCARE, UK, vol. 14, no. 9, 1 September 2010 (2010-09-01), pages 911-922, XP008176873, ISSN: 1472-8222, DOI: 10.1517/14728222.2010.504716

## Description

### Field of invention

The present invention relates to a conjugate comprising an anti-CCR6 antibody and a calcineurin inhibitor for use in a method for the treatment of an inflammatory disease. In addition, the invention relates to a method for identifying a subject likely to be resistant to steroid treatment, as well as a subject likely to benefit from treatment with a calcineurin inhibitor.

### Background to the invention

Autoimmune conditions are a significant burden to society and when grouped as a whole represent the third most common disease group in western populations after cancer and cardiovascular disease. The most commonly used treatments for autoimmune conditions are corticosteroids, which act broadly to suppress the immune system. However, in up to 30% of patients, steroid treatment is ineffective, with patients classed as steroid-refractory or steroid-resistant, the latter suffering the myriad side-effects of steroid treatment for no clinical benefit.

It is now well accepted that T helper (Th)17 cells represent a distinct CD4⁺ lineage and secretion of key cytokines IL-17A, IL-17F and IL-22 (1) are vital to their function in host defence against bacterial and fungal infections (1, 2). Also, the transcription factor, Retinoid-related orphan receptor (ROR)-yt has been demonstrated to be essential for their differentiation ((1, 3)). In humans, the majority of peripheral, circulating Th17 cells are thought to be derived from effector, memory T helper cells (CD4⁺CD45RO⁺) (4) with expression of the chemokine receptor, CCR6, highlighting those CD4+ T cells principally responsible for IL-17 production (5-7). However, more importantly, Th17 cells are also known to be key drivers in the development of autoimmune and inflammatory diseases such as uveitis, Behçet's disease, Vogt-Koyanagi-Harada (VKH) disease, multiple sclerosis, neuromyelitis optica, rheumatoid arthritis, psoriasis, inflammatory bowel disease (including Crohn's disease and ulcerative colitis), systemic lupus erythematosus, seronegative spondyloarthropies, Sjogren's syndrome, severe asthma, and allograft rejection post-organ transplantation (8-10; 27; 59-64).

Glucocorticoids remain the single most commonly used drug to treat all inflammatory diseases in man, including asthma, ulcerative colitis and psoriasis (11). However, up to a third of patients are sub-optimally responsive to glucocorticoid treatment (12, 13) and as such steroid refractory (SR) disease represents a significant clinical burden across specialities. Furthermore, these patients usually require high-dose, chronic treatment and the benefits are mitigated by a plethora of side effects, including centripetal obesity, diabetes mellitus, hypertension, and osteoporosis (14). Many proposed mechanisms to explain the SR paradigm have been described. However, there remains no precise reason as to why individuals present clinically as SR (15).

Glucocorticoids act through the glucocorticoid receptor (GR), regulating a broad spectrum of physiological processes which control glucose, protein, and fat metabolism, bone homeostasis, as well as immune responses mediated by both innate and adaptive immune cells, including CD4⁺ T cells and monocytes/macrophages (16). As such, many of the proposed mechanisms take this in to account. Overexpression of the dominant negative isoform of the GR, GR-β, which is incapable of binding glucocorticoid agonists (12, 17), genetic susceptibility and distinct SR syndromes attributed to specific genetic mutations and polymorphisms of the GR (12) as well as decreased synthesis of IL-10 by SR patients (18) have all been attributed to the SR phenotype. We have previously demonstrated that patients with SR diseases have a subpopulation of CD4⁺ T cells in their peripheral circulation that escape glucocorticoid suppression *in vitro* (19, 20). These SR T cells are restricted to the memory T cell population which displays an intermediate level of CD25 (20).

Recently, reports in human and murine asthma studies have demonstrated the involvement of Th17 cells in SR asthma (21-24). Stimulated human PBMCs from SR asthmatics have been shown to express greatly enhanced IL-17 production compared with non-SR asthmatics(24) while IL-17 stimulated PBMCs showed an increase in the dominant negative GR isoform, GR-β (22). Furthermore, in a murine model of asthma, adoptive transfer of Th17 cells were found to be both hyporesponsive to corticosteroid treatment and contributed to the disease pathology (21), while mice overexpressing RORyt developed SR airway inflammation (23), thus indicating Th17 cells may play a role in SR asthma (22). This becomes particularly relevant in those autoimmune diseases that display the SR phenotype. In uveitis (intraocular inflammation), diseases including Vogt-Koyanagi-Harada (VKH) and Behçet's disease, have been shown to be resistant to treatment with systemic corticosteroids (25) but are now known to have a Th17 bias (26, 27).

The majority of studies demonstrating that Th17 are SR have been carried out in experimental murine models and the glucocorticoid response of human Th17 cells has not been firmly established.

Clinically, amongst all the non-steroid interventions, calcineurin inhibitors have the strongest evidence base (28) and they are the second line treatment for steroid refractory patients (tacrolimus and cyclosporine are commercially available). Originally developed as an immunosuppressive for transplant patients, this T cell inhibitor principally acts to interfere with calcium-dependent signalling by forming a complex with cyclophilin, thereby preventing calcineurin from dephosphorylating and activating the transcription factor nuclear factor of activated T cells (NFAT), which ultimately prevents lymphocyte activation and transcription of downstream genes linked to an inflammatory immune response, such as IL-2 and IFN-γ (29). Therefore, inhibition of this pathway prevents inflammation. As such, calcineurin inhibitors are commonly used after organ transplantation to prevent inflammation, and resultant rejection of the transplanted organ. However, calcineurin inhibitor use is limited, due to inherent toxicity, particularly in the kidney.

At present, corticosteroids are the only therapeutic class approved by the FDA for the treatment of uveitis. However one third of patients with uveitis fail to achieve clinical remission with a tolerable systemic corticosteroid dose [51]. Such 'steroid refractory' (SR) disease is the cause of considerable morbidity, as affected individuals are subject to both the adverse sequelae of ongoing ocular inflammation and the systemic adverse effects of corticosteroids - including centripetal obesity, skin atrophy, osteoporosis, diabetes mellitus, hypertension, and mood disturbance[52]. Similar SR phenotypes are also well documented in a wide range of other inflammatory and lymphoproliferative conditions, and suboptimal steroid responses consequently represent a major disease burden across medical specialties [53-56].

However, it is standard practice to introduce 'second-line' immunosuppressive drugs in SR patients who are unable to achieve disease control on 10mg or less of oral prednisolone daily[57]. The most common drugs used for this purpose are calcineurin inhibitors (in particular cyclosporin A), antiproliferative drugs (including mycophenolate mofetil, azathioprine and methotrexate), and only if these fail, biologic therapies (predominantly anti-TNFalpha and Type 1 interferons)[58]. Among all non-steroid interventions used in clinical practice, cyclosporine A has the strongest evidence base [28]. However, its efficacy is greatly limited by associated renal toxicity and cardiovascular harm, which is also a feature of newer generation calcineurin inhibitors (including tacrolimus and voclosporin). The substantial nature of this nephrotoxicity has resulted in reluctance by clinicians to administer calcineurin inhibitors, despite their proven, efficacious immunosuppressive and anti-inflammatory record.

US 2002/094542 describes (separately) conjugates of cyclosporin A and anti-CD25 antibodies to treat autoimmune diseases.

EP 2,116,263 A1 describes conjugates of cyclosporin A and small molecules to treat autoimmune diseases.

WO 2010/065,962 describes antibodies directed at Th17 that may be conjugated and which may reduce or eliminate side effects of corticoid therapy.

WO 2013/102,042 describes antibody-drug conjugates comprising, for example, cyclosporin A or FK506 for treating autoimmune diseases.

WO 03/006,619 describes compositions that are useful for modifying calcineurin activity.

Steward-Tharp et al., Annals of the New York Academy of Sciences, 2010, pages 123-148 describe T cell-directed therapy for treating autoimmune diseases, including targeting Th17 cells.

Lacy et al., Americas Antibody Congress, mAbs, vol. 1, no. 6, 1 November 2009, pages 523-530 describe the development of antibody drug conjugates.

Kuypers et al., Clinical Transplantation, vol. 17, no. 3, 2003, pages 234-241 describe the use of an anti-CD25 monoclonal antibody and low-dose tacrolimus in renal transplant patients.

Hedrick *et al.,* Informa Healthcare, UK, vol. 14, no. 9, 2010, pages 911-922 describe CCR6 as a potential therapeutic target in psoriasis.

WO 2013/168,876 describes a method for monitoring the immune status of a subject after a transplant by measuring Th17 cells by means of anti-Th17 antibodies.

### Summary of invention

The present inventors have found that a subpopulation of T cells (Th17 cells) is preferentially susceptible to calcineurin inhibition in patients with inflammatory disease.

The inventors have further shown that this subpopulation of T cells is responsible for steroid resistance in patients with steroid-refractory inflammatory disease. The present invention is based on these findings and solves the problems of steroid resistance and the toxicity of calcineurin inhibitors by targeting a calcineurin inhibitor to this pathogenic T cell population by means of conjugation to a suitable specific antibody to enable selective inhibition of calcineurin in Th17 cells only. This allows the use of lower systemic doses of the calcineurin inhibitor, preventing unwanted kidney toxicity, systemic immunosuppression of other immune cell populations, as well as the clinical side effects seen with corticosteroids.

In a first aspect, the invention provides a conjugate comprising (i) an anti-CCR6 antibody and (ii) a calcineurin inhibitor for use in a method for the treatment of an inflammatory disease.

The inflammatory disease may be steroid resistant. The inflammatory disease may be an autoimmune disease or any T-cell mediated inflammatory disease. Examples of inflammatory diseases that may be treated according to the present invention include uveitis, Behçet's disease, Vogt-Koyanagi-Harada (VKH) disease, multiple sclerosis, neuromyelitis optica, rheumatoid arthritis, psoriasis, inflammatory bowel disease (including Crohn's disease and ulcerative colitis), systemic lupus erythematosus, seronegative spondyloarthropies, Sjogren's syndrome, and severe asthma. In some embodiments, the subject to be treated may be post-organ transplantation, such that treatment with the conjugate described herein reduces the risk of steroid resistant allograft rejection. Preferably, the inflammatory disease is uveitis.

The calcineurin inhibitor linked to the anti-CCR6 antibody is preferably tacrolimus, cyclosporin A (CsA), or voclosporin. Most preferably, the calcineurin inhibitor linked to the anti-CCR6 antibody is tacrolimus.

In a preferred embodiment, the conjugate comprises an anti-CCR6 antibody linked to tacrolimus or CsA.

In a particularly preferred embodiment, the invention provides a conjugate comprising an anti-CCR6 antibody linked to tacrolimus or CsA for use in a method for the treatment of uveitis.

In any of the embodiments described herein, the antibody may, for example, be conjugated to the beta-hydroxyl group of the butenyl-methyl-L-threonine residue of CsA.

Prior to treatment with the conjugate, expression of IL-17 may be determined in a sample of T cells obtained from the subject, such that a subject is selected for treatment with the conjugate if the sample of T cells obtained from said subject expresses IL-17 at a higher level than a reference sample of T cells or at a higher level than a reference value (e.g. concentration or amount) of IL-17. The reference sample of T cells is preferably obtained from a normal, healthy subject.

Alternatively, or in addition, prior to treatment with the conjugate, expression of CCR6 may be determined in a sample of T cells obtained from the subject, such that a subject is selected for treatment with the conjugate if the sample of T cells obtained from said subject expresses CCR6 at a higher level than a reference sample of T cells or at a higher level than a reference value (e.g. concentration or amount) of CCR6. The reference sample of T cells is preferably obtained from a normal, healthy subject.

The invention provides a method for identifying a subject likely to be resistant to steroid treatment, wherein the method comprises the step of determining expression of CCR6 in a sample of T cells obtained from the subject, such that expression of CCR6 in the sample at a higher level than a reference sample of T cells or at a higher level than a reference value (e.g. amount or concentration) of CCR6 indicates that the subject is likely to be resistant to steroid treatment. The reference sample of T cells is preferably obtained from a normal, healthy subject.

The subject has preferably been diagnosed with an inflammatory disease, such as an autoimmune disease. Examples of inflammatory diseases that may be diagnosed according to the present invention include uveitis, Behçet's disease, Vogt-Koyanagi-Harada (VKH) disease, multiple sclerosis, neuromyelitis optica, rheumatoid arthritis, psoriasis, inflammatory bowel disease (including Crohn's disease and ulcerative colitis), systemic lupus erythematosus, seronegative spondyloarthropies, Sjogren's syndrome, and severe asthma. Alternatively, the subject may be post-organ transplantation. Most preferably, the inflammatory disease is uveitis.

The invention provides a method for identifying a subject likely to benefit from treatment with a calcineurin inhibitor, wherein the method comprises the step of determining expression of CCR6 in a sample of T cells obtained from the subject, such that the expression of CCR6 in the sample at a higher level than a reference sample of T cells or at a higher level than a reference value (e.g. amount or concentration) of CCR6 indicates that the subject is likely to benefit from treatment with the calcineurin inhibitor. The reference sample of T cells is preferably obtained from a normal, healthy subject.

The calcineurin inhibitor to be used for treatment is for use as a conjugate comprising (i) an anti-CCR6 antibody and (ii) a calcineurin inhibitor.

Preferably, the subject has previously been diagnosed with an inflammatory disease. More preferably the inflammatory disease is steroid-resistant. The inflammatory disease may be an autoimmune disease. Examples of inflammatory diseases that may be diagnosed according to the present invention include uveitis, Behçet's disease, Vogt-Koyanagi-Harada (VKH) disease, multiple sclerosis, neuromyelitis optica, rheumatoid arthritis, psoriasis, inflammatory bowel disease (including Crohn's disease and ulcerative colitis), systemic lupus erythematosus, seronegative spondyloarthropies, Sjogren's syndrome, and asthma. In some embodiments, the subject may be post-organ transplantation, such that treatment with the conjugate described herein reduces the risk of steroid resistant allograft rejection. Most preferably, the inflammatory disease is uveitis.

The calcineurin inhibitor is preferably tacrolimus, cyclosporin A (CsA), or voclosporin. Most preferably, the calcineurin inhibitor is tacrolimus.

CCR6 is also expressed on non-Th17 memory T cells and hence an antibody-drug conjugate which uses calcineurin inhibition to specifically inhibit T cell function in CCR6-expressing cells will in theory suppress the wider memory pool of T cells including, but not limited to, Th17 cells. This would be expected to selectively neutralise the memory adaptive immune response, while leaving the naive immune response intact, leaving the recipient immunocompetent, while avoiding harm to non-immune tissues. The applicability of this invention therefore extends beyond steroid resistance to all T-cell mediated diseases, including but not limited to multiple sclerosis, rheumatoid arthritis, inflammatory bowel disease, psoriasis and asthma.

Thus, the application provides a conjugate comprising an anti-CCR6 antibody and a calcineurin inhibitor, such as tacrolimus, cyclosporin A or voclosporin for use in a method for the treatment of T-cell mediated inflammatory disease, such as multiple sclerosis, rheumatoid arthritis, inflammatory bowel disease, psoriasis and asthma.

These and other aspects of the invention are described in further detail below.

### Brief description of drawings

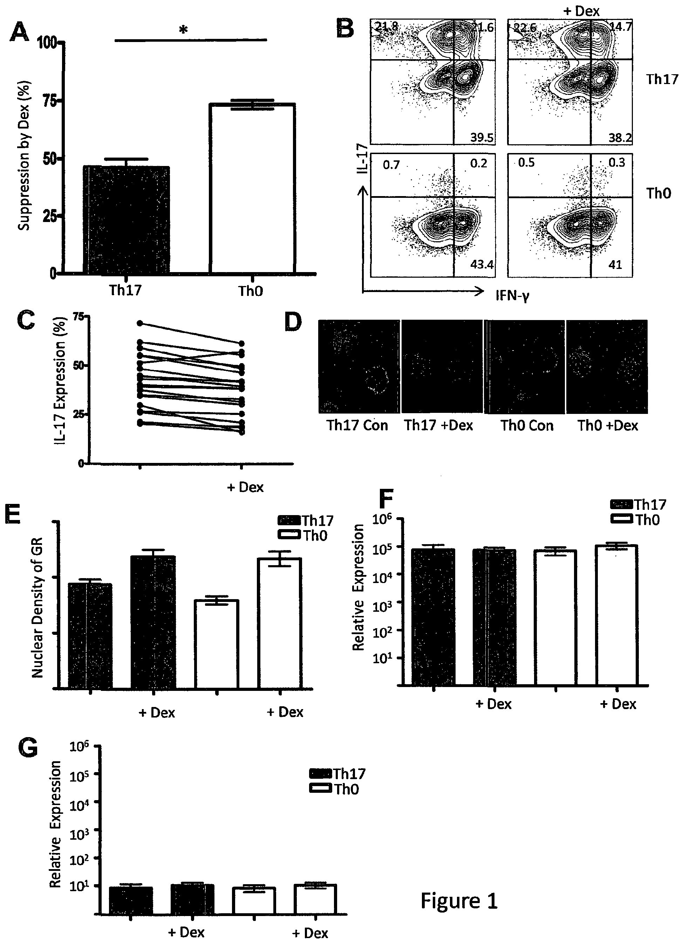

Figure 1 shows that human Th17 cells are resistant to glucocorticoid suppression. (A) Following *in vitro* culture for 7 days, CD4⁺CCR6⁺Th17 and CD4⁺CCR6⁻Th0 cells were treated with Dex for 24 hours and subjected to the T cell proliferation assay. The percentage of proliferation reduction was calculated (n=3, p=0.03). (B) Representative flow cytometric analysis of Th17 and Th0 polarized cells treated with or without Dex, stimulated with PMA and ionomycin for 4 hours followed by flow cytometric analysis of IL-17 and IFN-γ expression, as indicated. (C) Combined data indicating individual sample analysis for IL-17 expression from Th17 cells following Dex treatment. n=18. (D) Representative examples of GR fluorescent staining as detected by confocal microscopy, before and after 30 minutes Dex treatment in Th17 and Th0 cells. (E) Relative nuclear density of GR expression, data are representative of at least three independent experiments. (F) Relative expression of total GR in Th17 and Th0 cells with or without 30 minutes Dex treatment. (G) Relative expression of GR-β isoform in Th17 and Th0 cells with or without 30 minutes Dex treatment. All data are representative of 3 or more independent experiments and indicate the mean ± SEM.

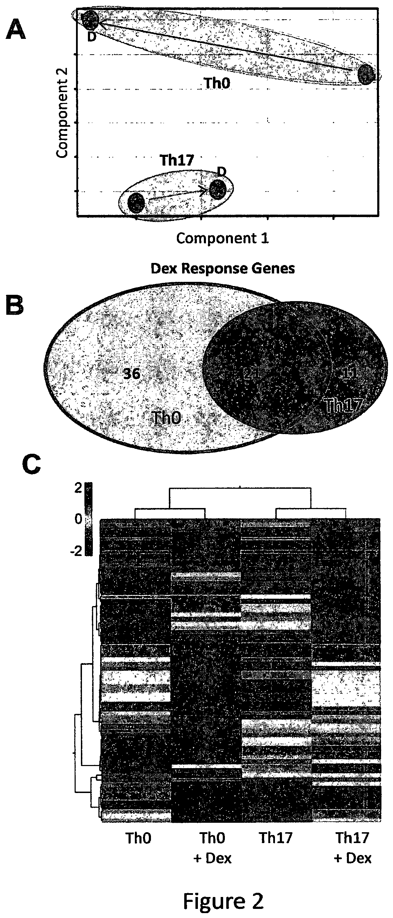

Figure 2 shows that genome-wide expression profiles reveal a significant difference between human Th17 and Th0 cells following glucorticoid treatment. (A) Principle component analysis on genes with at least two fold changes between any two of the four conditions (untreated and Dex treated Th17 and Th0 cell; D: Dex treated). (B) Venn Diagram illustrating the different sets of Dex response genes (with at least two fold change between Th17 vs Th17+Dex, or Th0 vs Th0+-Dex, and p<0.05) between Th17 and Th0 cells. (C) Hierarchical clustering of all 68 Dex response genes shown in Fig. 2B.

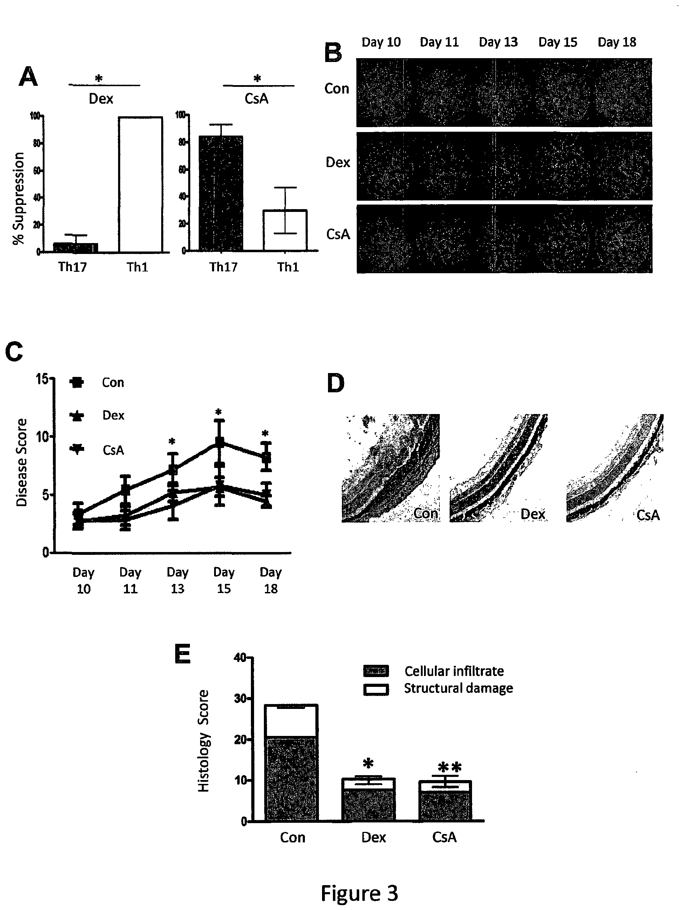

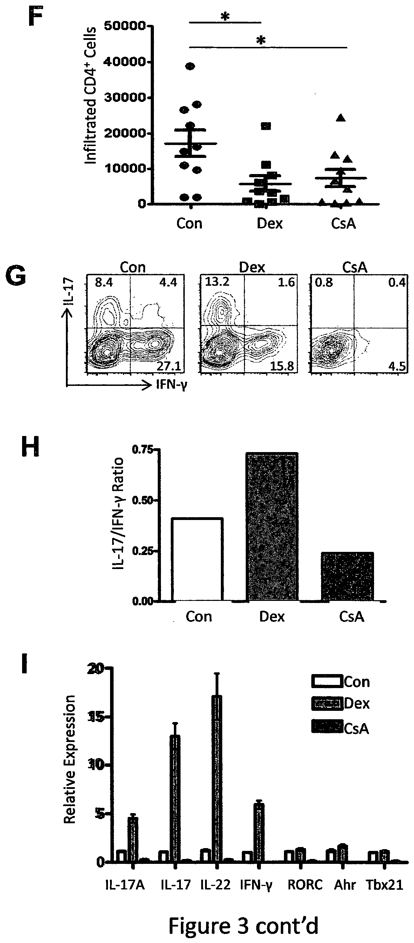

Figure 3 shows that murine glucocorticoid resistant Th17 cells are controlled by calcineurin inhibition. (A) Naive CD4+ T cells from OT-II mice were cultured and activated under Th17 or Th1 polarizing conditions for 14 days, followed by Dex or CsA treatment for 48 hours. Cells then were subjected to the T cell proliferation assay and the percentage of proliferation suppression was calculated (n=5, p<0.05. (B) B10.RIII mice were immunized with RBP-3₁₆₁₋₁₈₀ and pertussis toxin and from Day 10 post-immunization, Topical endoscopic fundal imaging (TEFI) was used to follow the clinical features of the retina. On Day 11, 13, 15, and 17 post-immunization, mice were either treated with Dex or CsA or were left untreated (Control). Photographs are representative of a dilated, right eye from one mouse from each group. (C) Disease scores from each mouse following immunization for EAU and treatment with or without Dex or CsA on Day 11, 13, 15, and 18 post-immunization (n= 9 for each group, p<0.05). Both right and left eyes were scored and averaged and data are representative of 3 independent experiments. (D) Enucleated eyes were snap frozen on Day 18 following treatment with or without Dex or CsA, cryosectioned and stained with haemotoxylin and eosin (H&E). Representative 12-pm retinal sections from each group are illustrated. (E) Summary of histological retinal cellular infiltrate and structural damage from EAU mice treated with or without Dex or CsA on Day 18 was scored (n=3, p<0.05). (F) On Day 18, following treatment with or without Dex or CsA, EAU mice were sacrificed, eyes were enucleated and retina and ciliary body excised and digested. The total number of CD4⁺ T cells per eye was measured by flow cytometry (n=10 in each group, p<0.05). (G) As for Fig 3F, following retina and ciliary body digestion, eyes from each group were pooled together, CD4+ T cells enriched, stimulated with PMA and ionomycin for 4 hours, and then stained for intra cellular IL-17 and IFN-γ expression (n=9 in each group). (H) The ratio of IL-17 / IFN-γ was calculated from the data generated from Fig 3G. (I) Relative expression of genes, detected by Real-time PCR, in retina infiltrating CD4⁺ T cells from the eyes of EAU mice treated with or without Dex or CsA on Day 18 (n=9 for each group). All data are represented as mean ± SEM.

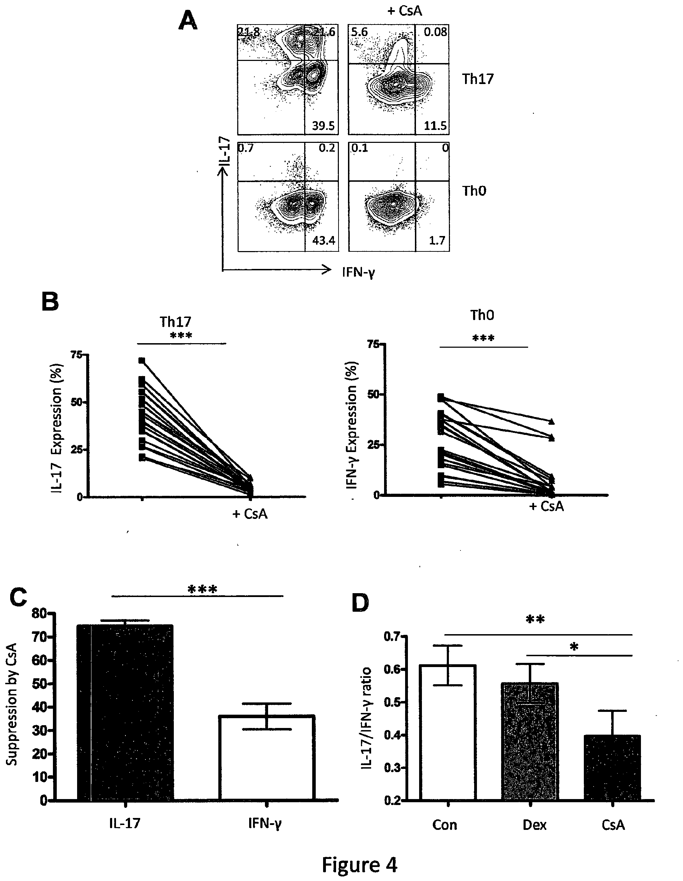

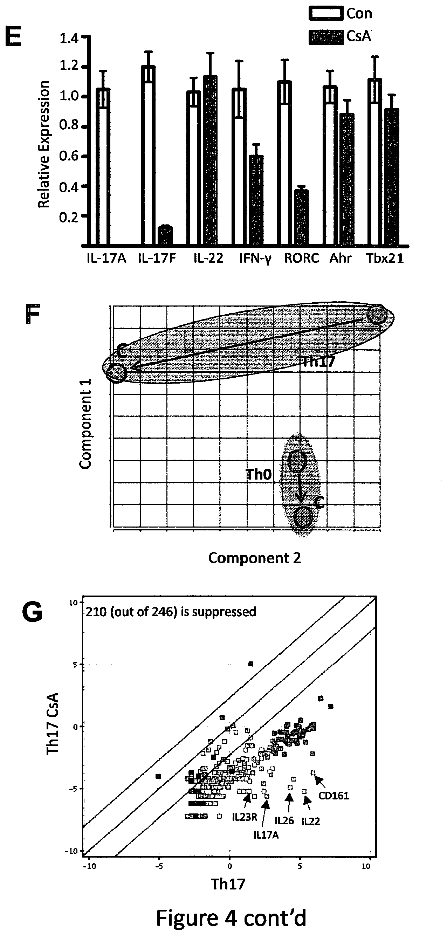

Figure 4 shows that calcineurin inhibition preferentially suppressed human Th17 cells. (*A*) *In vitro* Th17 and Th0 polarized cells were treated with CsA for 24 hours, stimulated with PMA and ionomycin for 4 hours followed by flow cytometric analysis of IFN-γ and IL-17 expression. (*B*) Summary of the frequency of CD4⁺IL-17⁺ cells in Th17 cultures (left hand graph) and CD4⁺IFN-γ⁺ with or without CsA treatment (n=21, p<0.0001). (*C*) Summary of the percentage of reduction of the IL-17 or IFN-γ single positive cells in Th17 cultures following 24 hours CsA treatment (n=20, p<0.0001). (D) The ratio of IL-17 / IFN-γ expression from Th17 cultures following 24 hours of Dex or CsA treatment (n=20, p<0.05)) (*E*) Relative expression of genes, detected by Real-time PCR, in human Th17 cultures with or without CsA treatment (n=3). (F) Principle component analysis on genes with at least two fold changes between any two of the four conditions (untreated and CsA treated Th17 and Th0 cell; C: CsA treated). (G) Scatter plot of suppression of gene expression in Th17 cultures treated with (Y-axis) or without (X-axis) CsA (as measured by RNA-sequencing). Absolute expression was plotted on a log2 scale colour coded from low to high expression. Genes that are up or down in each group are highlighted (n=2). All data are represented as mean ± SEM.

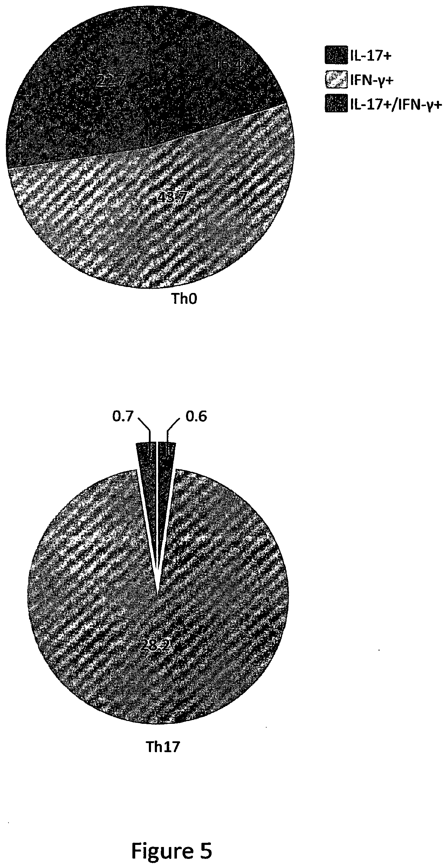

Figure 5 shows pie charts representing phenotype of Th17 and Th0 polarized cells. Compiled data from the end of two weeks culture illustrating the overall distribution of IL-17 and IFN-γ single positive cells and IL-17/IFN-γ double positive cells within the Th17 and Th0 subsets (n=23).

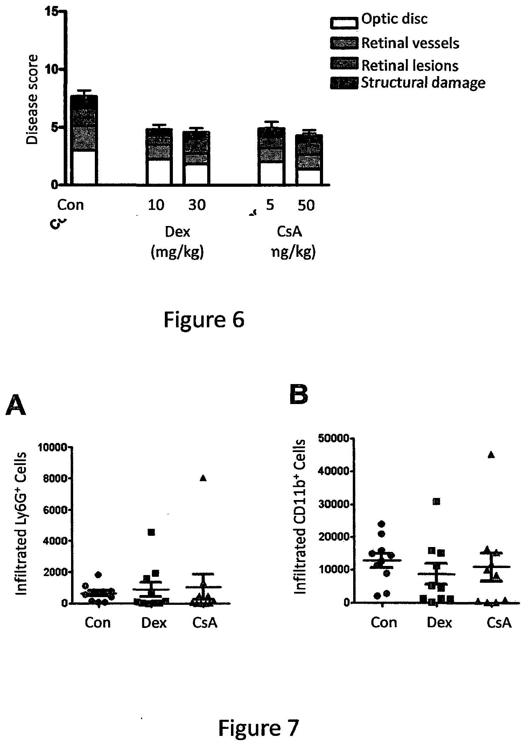

Figure 6 provides a day 18 summary of TEFI disease score of mice immunized for EAU and then treated with either Dex (10 or 30mg/kg), CsA (5 or 50mg/kg) or left untreated (Control). The optic disc/retinal vessel damage, as well as the size of retinal lesion and retinal structure damage were scored for each eye from each mouse and then averaged (n=6 for each group). Data are represented as mean ± SEM.
Figure 7 shows a characterisation of inflammatory cell infiltrate from the eyes of mice immunized for EAU and treated with or without Dex or CsA. On Day 18, following treatment, mice were sacrificed, eyes enucleated and retina and ciliary body excised and digested. (*A*) Total number of CD45⁺CD11b⁺Ly6G⁺ neutrophils were measured by flow cytometry (n=10 in each group). (*B*) Total number of CD45⁺CD11b⁺ myeloid cells were measured by flow cytometry (n=10 in each group). Data are represented as mean ± SEM.

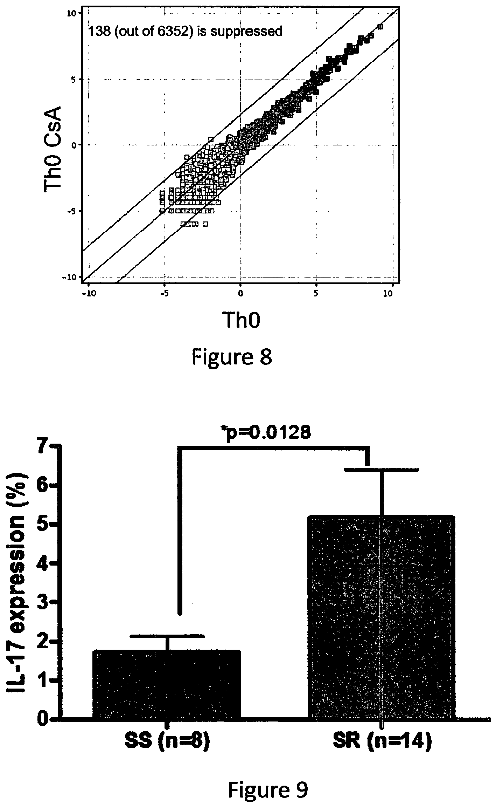

Figure 8 shows a scatter plot of suppression of gene expression in Th0 cultures treated with (Y-axis) or without (X-axis) CsA. Absolute expression was plotted on a log2 scale colour-coded from low to high expression. Genes that are up or down in each group are highlighted (n=2).
Figure 9 shows IL-17 expression by CD4+ cells from steroid sensitive (SS) and steroid resistant (SR) uveitis patients' peripheral blood following anti-CD3/anti-CD28 T cell receptor ligation.

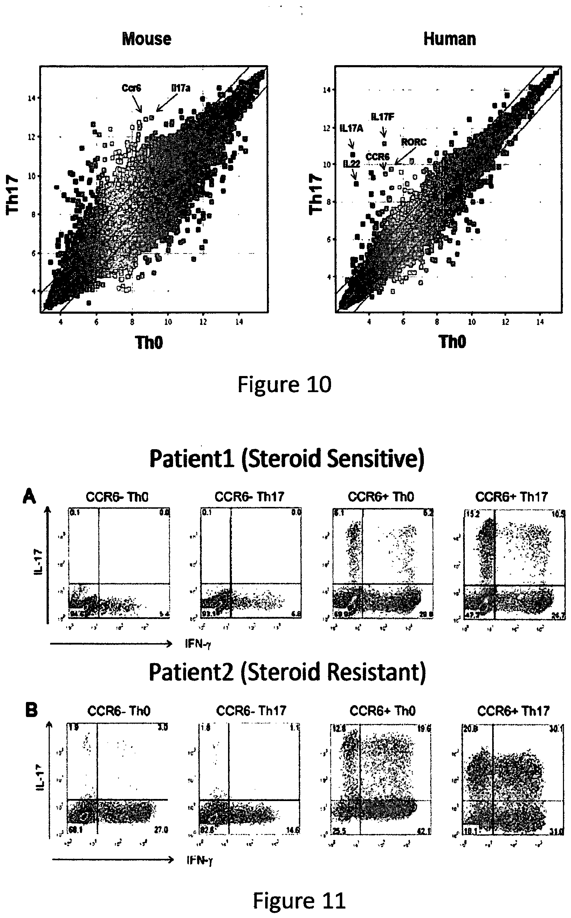

Figure 10 shows murine and human CD4+ T cells that were polarised (Th17) in the presence of cytokines or remained unpolarised (Th0). For the murine analysis (left hand scatter plot), 4 CD4+ T cell cultures (n=2 for each Th17/Th0) were analysed and for the human analysis, 8 CD4+ cells cultures (n=4 for each Th17/Th0). The highest ranking probes are highlighted and labelled.
Figure 11 shows a representative example of a steroid sensitive uveitis patient (patient 1, top row) and a steroid resistant uveitis patient (patient 2, bottom row) CD4+ T cells that have been sorted based on CCR6 expression into CCR6+ or CCR6- and cultured for 2 weeks with (Th17) or without (Th0) polarising cytokines.

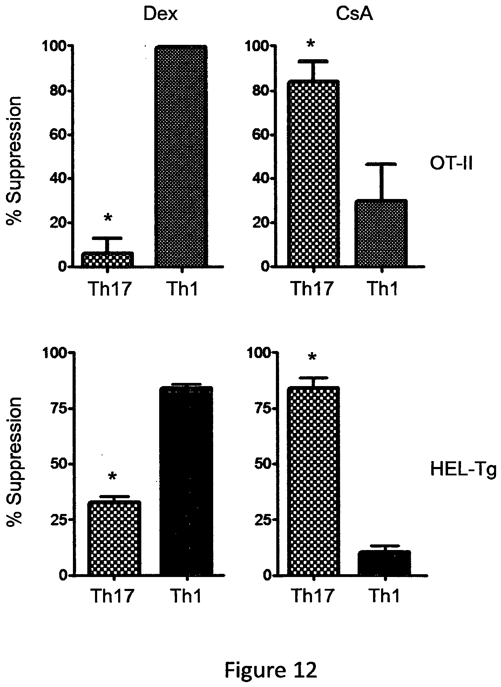

Figure 12 shows the naive CD4⁺ T cells from HEL and OT-II-Transgenic mice that were cultured and activated under Th17 or Th1 polarizing conditions for 14 days, followed by Dex or CsA treatment for 48 hours. Cells then were subjected to the T cell proliferation assay and the percentage of proliferation suppression was calculated (n=5, p<0.05).

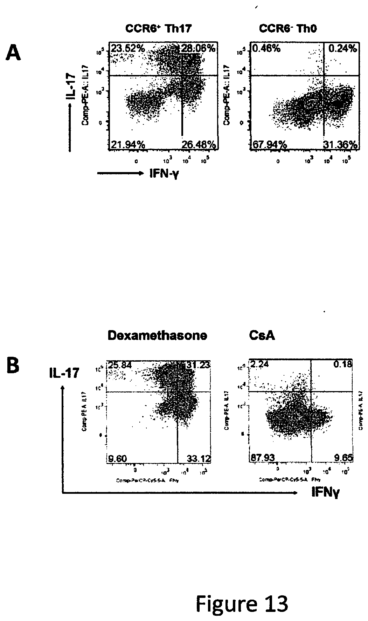

Figure 13A is a representative example of the expression profile of CCR6+Th17 and CCR6-Th0 cells following 2 weeks culture. The canonical Th1 cytokine (IFN-γ) and Th17 cytokine (IL-17) is labelled. Figure 13B is a representative example of the expression profile of the CCR6+Th17 cells treated with dexamethasone or CsA for 24 hours. IL-17 expression is not suppressed by Dex, but almost completed ablated by CsA.

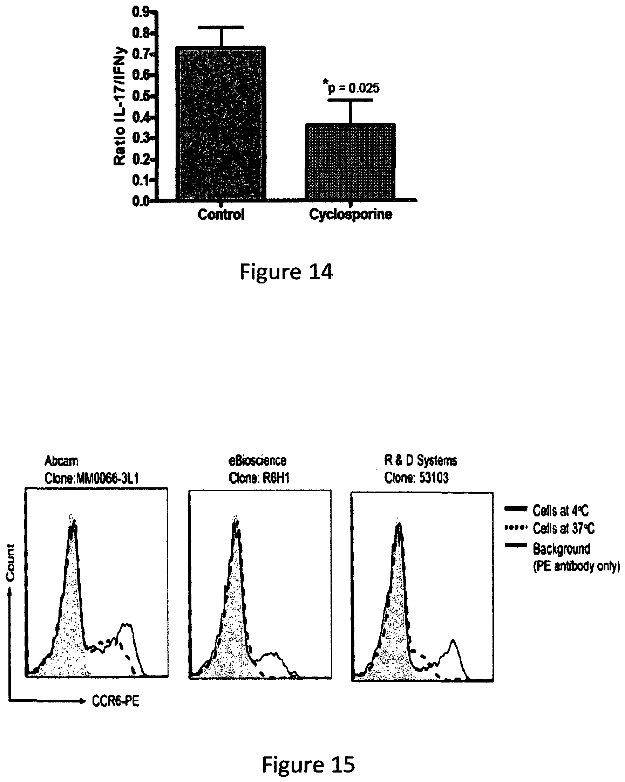

Figure 14 shows the ratio of IL-17 to IFN-γ after cyclosporine treatment, demonstrating that IL-17 is preferentially inhibited relative to IFN-γ (n=10 healthy volunteers).
Figure 15 shows the internalisation of the CCR6 receptor following receptor ligation. Three commercial monoclonal antibodies (all different clones) were tested for internalisation following a brief incubation at 37°C.

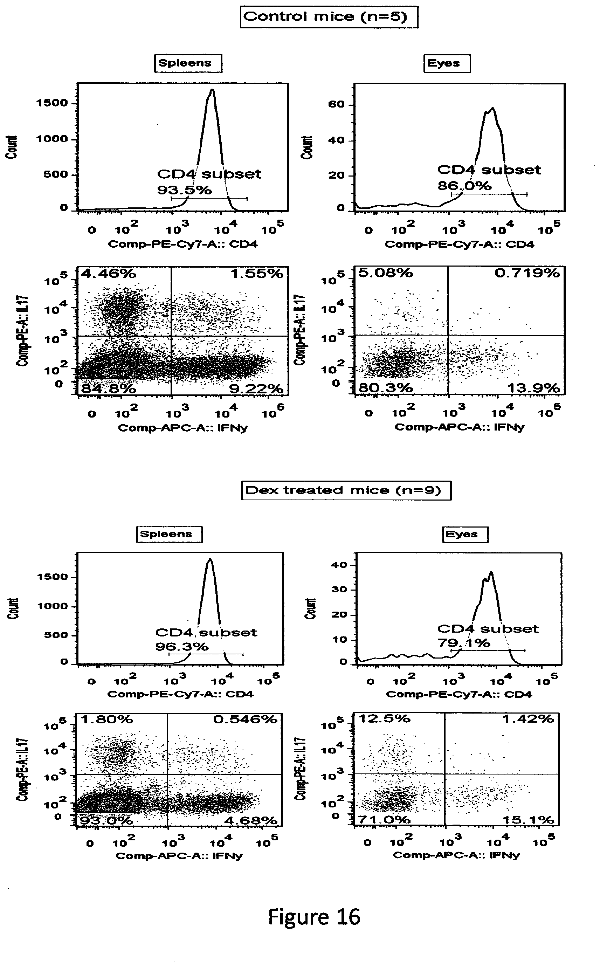

Figure 16 shows the results from the B10.RIII mice that were immunized with RBP-3₁₆₁₋₁₈₀ and pertussis toxin. On Day 11, 13, 15, and 17 post-immunization mice were either treated with Dex or were left untreated (Control). On Day 18, following treatment with or without Dex, mice were sacrificed, eyes were enucleated, retina and ciliary body excised and digested. Eyes from each group were pooled together, CD4⁺ T cells were enriched, stimulated with PMA and ionomycin for 4 hours, and then stained for intracellular IL-17 and IFN-γ expression (n=9 in each group). The spleens from each animal in both groups were also harvested, mechanically digested into a single cell suspension, CD4⁺ T cells were enriched, stimulated with PMA and ionomycin for 4 hours, and then stained for intracellular IL-17 and IFN-γ expression (n=9 in each group).

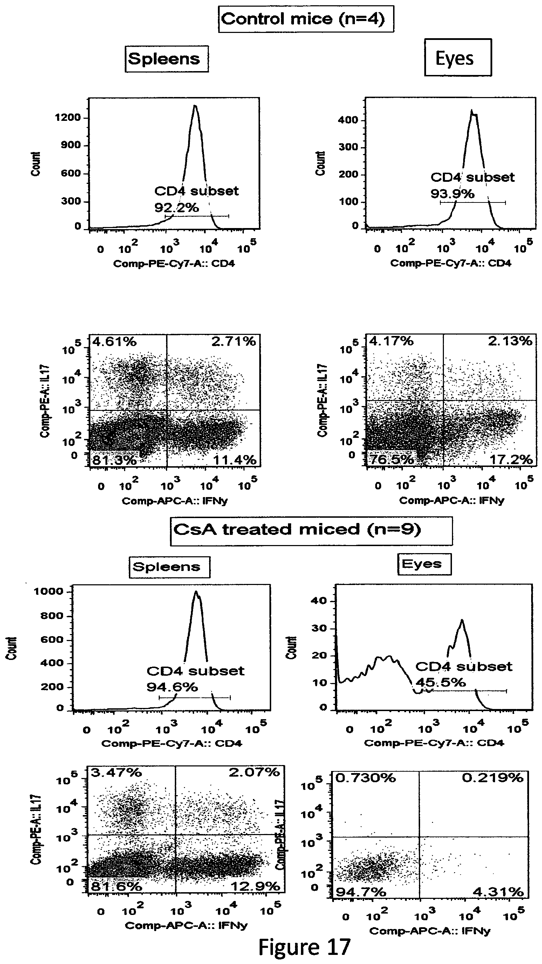

Figure 17 shows the results from the B10.RIII mice that were immunized with RBP-3₁₆₁₋₁₈₀ and pertussis toxin. On Day 11, 13, 15, and 17 post-immunization mice were either treated with CsA or were left untreated (Control). On Day 18, following treatment with or without CsA, mice were sacrificed, eyes were enucleated, retina and ciliary body excised and digested. Eyes from each group were pooled together, CD4⁺ T cells were enriched, stimulated with PMA and ionomycin for 4 hours, and then stained for intracellular IL-17 and IFN-γ expression (n=9 in each group). The spleens from each animal in both groups were also harvested, mechanically digested into a single cell suspension, CD4⁺ T cells were enriched, stimulated with PMA and ionomycin for 4 hours, and then stained for intracellular IL-17 and IFN-γ expression (n=9 in each group).

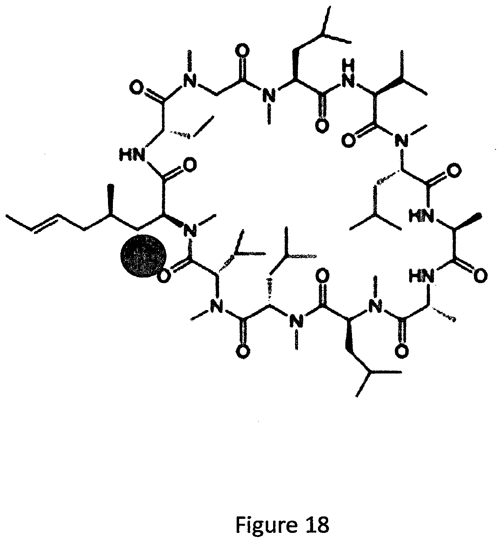

Figure 18 shows the chemical structure of Cyclosporine A to determine the specific-site for antibody conjugation. A desk based analysis showed that it has only one natural nucleophilic site for conjugation.

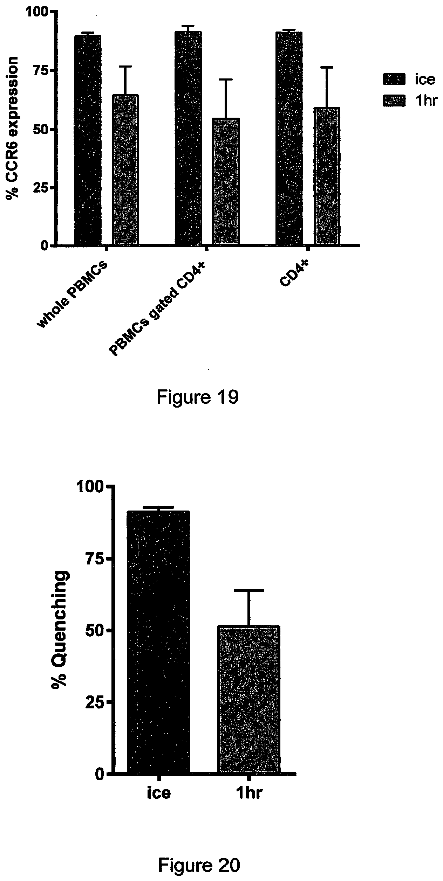

Figure 19 shows internalisation of CCR6 in human CD4+ cells in peripheral blood mononuclear cell (PBMC) culture and isolated CD4+ T cells. Data represent the mean of three normal volunteers, with error expressed as standard deviation.
Figure 20 shows internalisation of anti-CCR6 monoclonal antibodies (mAbs) on human CD4+ T cells using Alexa-488 quenching assay. Anti-CCR6 MAbs were conjugated with the Alexa-488 fluorochrome. This was then used to label extracellular CCR6 on isolated human CD4+ T cells. Following incubation under tissue culture conditions, cells were incubated with or without anti-Alexa-488 mAb. The proportion of quenching, which inversely corresponds to the proportion of internalised CCR6 (in complex with anti-CCR6-Alexa 488), was then measured using flow cytometry. A reduction in % quenching from the control sample (ice) indicates that the CCR6:mAb complex has become internalised. Data represent the mean of five normal volunteers, with error expressed as standard deviation.

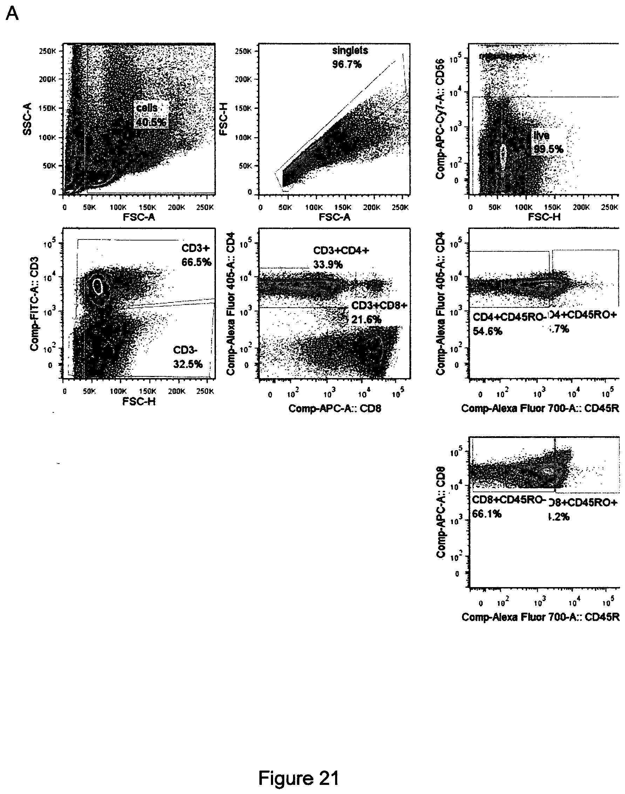

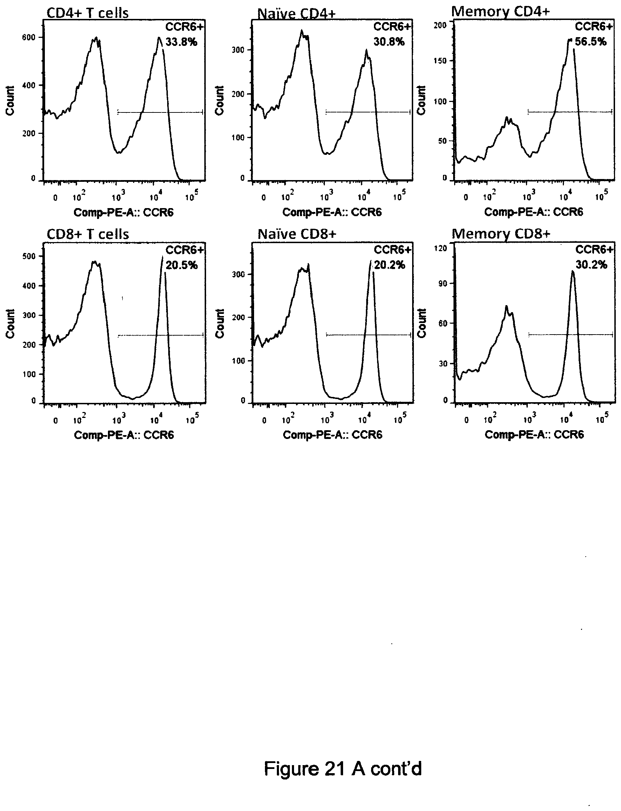

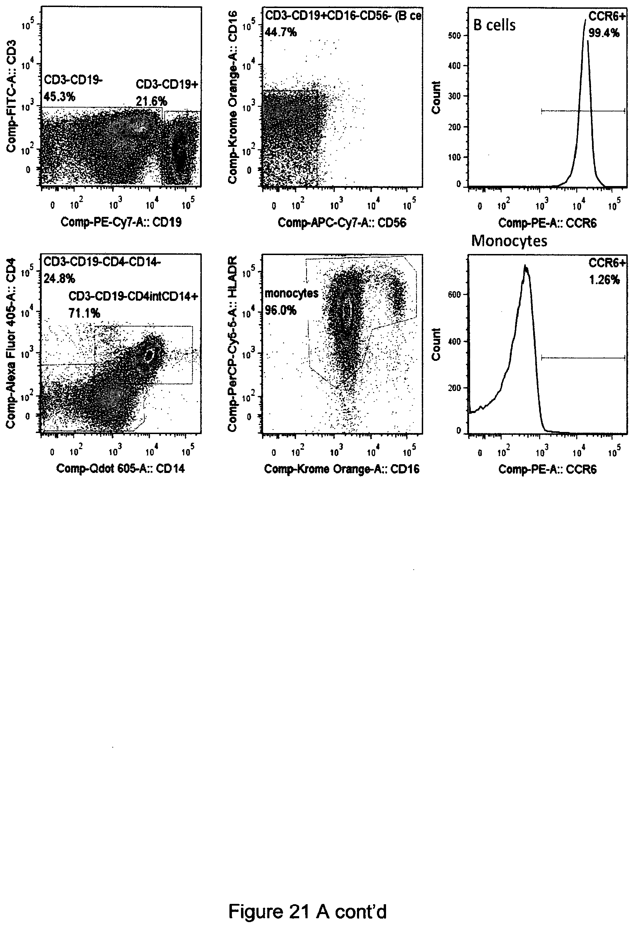

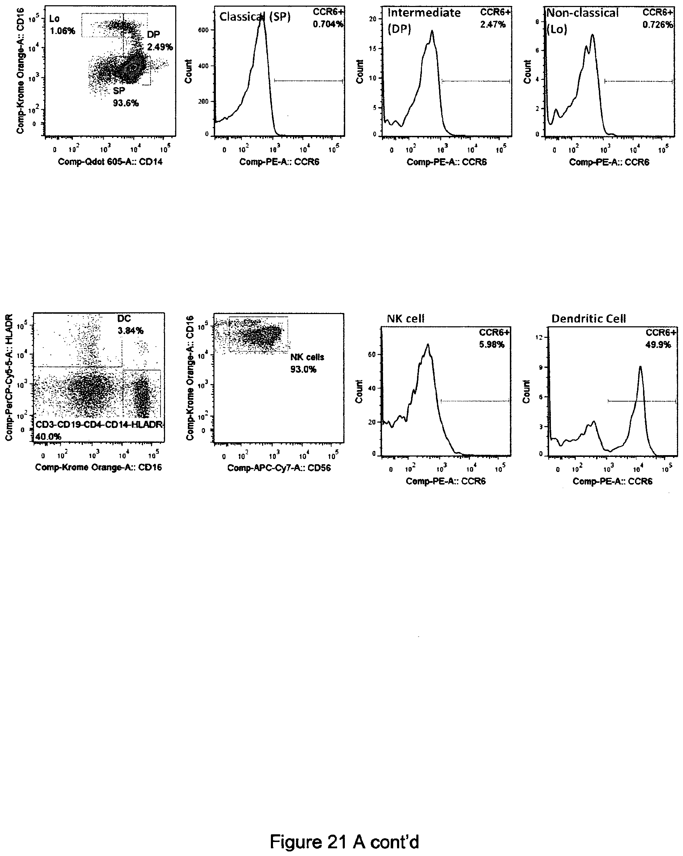

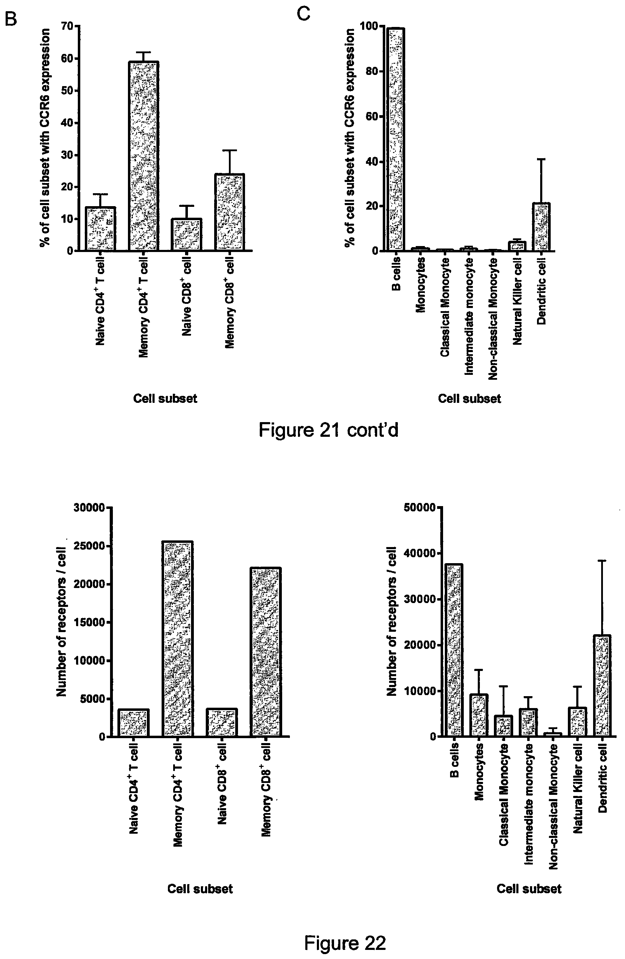

Figure 21 shows CCR6 expression on immune cell subsets in the blood of human normal volunteers. Isolated peripheral blood mononuclear cells were surface stained with a selection of markers as shown in (A). Each cell subset was assessed for the percentage of CCR6 expression, summarised in (B) and (C). Data represents the mean expression of four normal volunteers, with error expressed as standard deviation.
Figure 22 shows the absolute number of CCR6 receptors on immune cell subsets in the blood of human normal volunteers. Isolated peripheral blood mononuclear cells were surface stained with a selection of markers as shown in Figure 21A. The number of cell surface CCR6 receptors for each immune cell type was quantified against a standard curve from mean fluorescence intensity as determined by flow cytometry. Data represent the mean expression of one to three normal volunteers, with error, where shown, expressed as standard deviation.

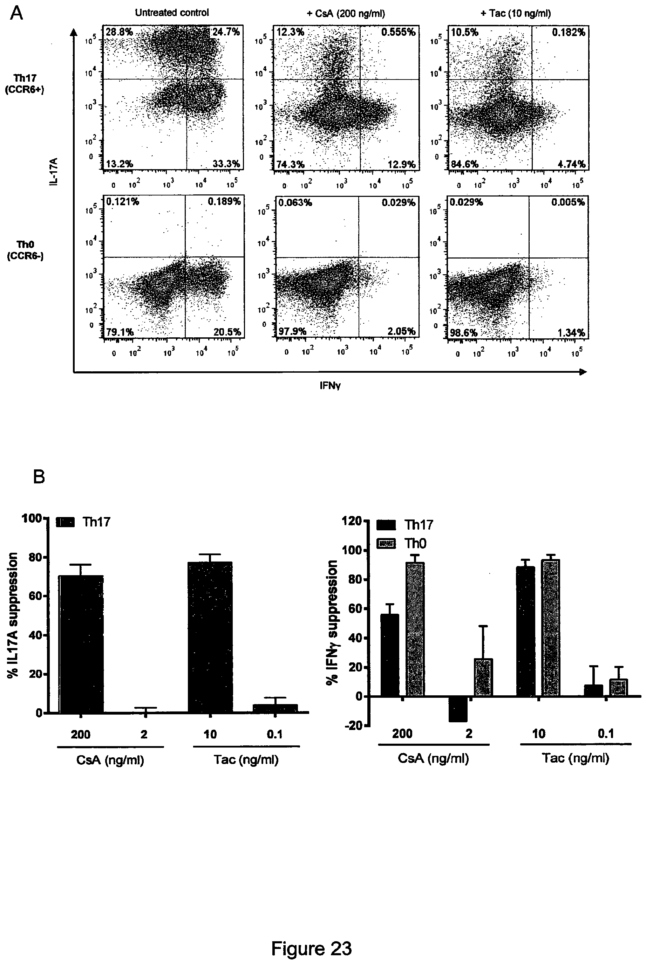

Figure 23 shows suppression of IL-17A in human Th17 (CCR6+) cells and IFNγ cytokine production in human Th0 (CCR6⁻) and Th17 cells. CCR6 positive and CCR6 negative CD4⁺ T cells were purified and cultured in the presence of polarising cytokines for two weeks. Cells were incubated for 24 hrs in the absence or presence of Cyclosporine A (CsA) or Tacrolimus (Tac). Intracellular cytokines were quantified by flow cytometry (A) and percentage suppression calculated in relation to an untreated control (B). Data represents the mean expression of three to four normal volunteers, with error expressed as standard deviation.

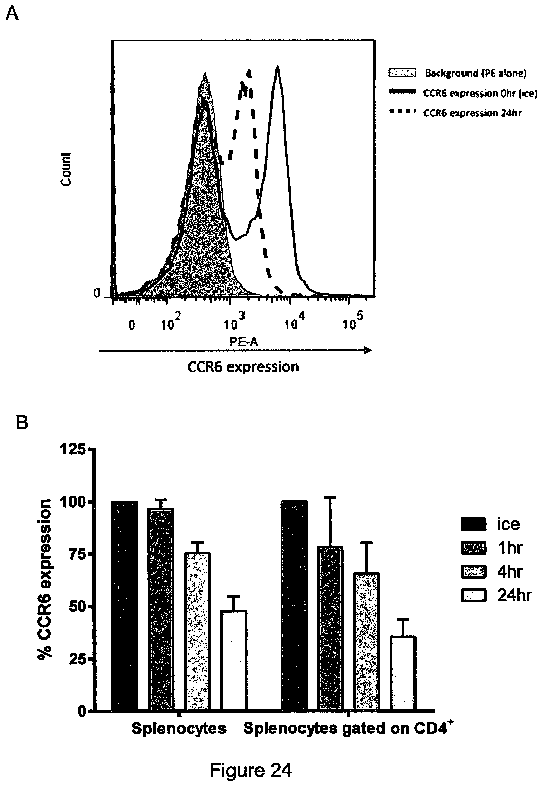

Figure 24 shows internalisation of CCR6 on CD4⁺ T cells in whole splenocyte populations in B10.RIII mice. Splenocytes from B10.RII mice were incubated with murine anti-CCR6 mAb, before detection of surface retained CCR6:anti-CCR6 mAb complex using a secondary mAb conjugated to R-Phycoerythrin (PE). Data represent the mean expression of spleens from two to four mice, with error expressed as standard error of mean.

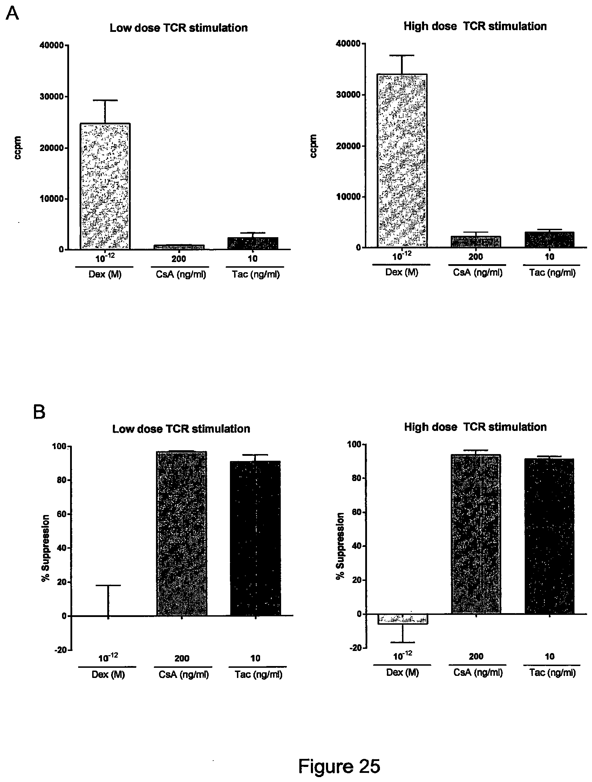

Figure 25 shows inhibition of T cell proliferation by the calcineurin inhibitors cyclosporine A and tacrolimus. Isolated CD4⁺ T cells were incubated under tissue culture conditions with low or high dose T-cell receptor stimulation in the presence of low dose dexamethasone (Dex) or high dose cyclosporine A (CsA) or tacrolimus (Tac). Proliferation was measured by tritiated thymidine incorporation (ccpm). Data represent the mean of two normal volunteers, with error expressed as standard deviation.

### Detailed description

The present inventors have identified a subpopulation of T cells (Th17 cells) that are preferentially susceptible to calcineurin inhibition and resistant to suppression with corticosteroids. The inventors have further shown that this subpopulation of T cells is prevalent in patients with steroid resistant inflammatory disease. Based on these findings, the present invention relates broadly to the use of a conjugate that specifically targets a calcineurin inhibitor to this Th17 subpopulation of T cells to treat an inflammatory disease. By specifically targeting the calcineurin inhibitor in this way, the problems of steroid resistance and the toxicity of calcineurin inhibitors are solved. This allows a lower systemic dose of the calcineurin inhibitor to be used, reducing the risk of kidney toxicity, systemic immunosuppression of other immune cell populations and the clinical side effects seen with corticosteroids.

As set out above, the present invention relates to a conjugate comprising (i) an an anti-CCR6 antibody and (ii) a calcineurin inhibitor for use in a method for the treatment of an inflammatory disease.

As the conjugate targets the calcineurin inhibitor specifically to Th17 cells, the calcineurin inhibitor does not affect renal cells, thereby avoiding the serious side effect of toxicity to the kidney observed with general calcineurin inhibitor use. The specific targeting of Th17 cells in Th17-driven inflammatory disease also ensures that it is the pathogenic cells causing the disease that are inhibited, while allowing other immune cells to remain active and able to defend against infection. This reduces the risk of unwanted systemic immunosuppression that would occur during general calcineurin inhibition or successful steroid treatment. In addition, patients are not usually resistant to calcineurin inhibition, whereas around 30% of patients are resistant to steroid treatment. Therefore, the present invention also overcomes the problem of steroid resistance in patients with inflammatory disorders.

The conjugate for use in the invention comprises an antibody that binds to the Th17 cell surface marker CCR6. This allows selective targeting of the calcineurin inhibitor to Th17 cells.

The term "antibody" is used in its broadest sense and specifically covers monoclonal antibodies, polyclonal antibodies, dimers, multimers, multispecific antibodies (e.g. bispecific antibodies), intact antibodies (also described as "full-length" antibodies) and antibody fragments, provided they exhibit the desired biological activity, i.e. the ability to bind to a Th17 cell surface marker. Examples of antibody fragments include Fab, Fab', F(ab')₂ and scFv fragments and diabodies. The antibody may be murine, human, humanised, chimeric, or derived from other species.

Most preferably, the antibody is a monoclonal anti-CCR6 antibody.

The antibody that binds to a Th17 cell surface marker is an anti-CCR6 antibody (e.g. a monoclonal anti-CCR6 antibody) that is internalised upon binding to CCR6.

As used herein, "binds to a Th17 cell surface marker" is used to mean that the antibody binds to the Th17 cell surface marker with a higher affinity than a non-specific partner, such as bovine serum albumin (BSA). The antibody preferably binds to the Th17 cell surface marker with a high affinity. For example, in some embodiments, the antibody can bind to the Th17 cell surface marker with a dissociation constant (k_{D}) of equal to or less than about 10⁻⁶ M, i.e. equal to or less than about 1 x 10⁻⁶M, equal to or less than about 1 x 10⁻⁷M, equal to or less than about 1 x 10⁻⁸M, equal to or less than about 1 x 10⁻⁹M, equal to or less than about 1 x 10⁻¹⁰M, equal to or less than about 1 x 10⁻¹¹M, or equal to or less than about 1 x 10⁻¹²M. Methods of determining k_{D} are well known in the art and include, for example, the use of a BIAcore™ optical biosensor that uses surface plasmon resonance for monitoring macromolecular interactions.

CCR6 is also known as CD196 and its Swissprot reference is P51684. CD25 is also known as the alpha chain of the IL-2 Receptor and its Swissprot reference is P01589.

The antibody preferably binds to CCR6 with a k_{D} of equal to or less than about 10⁻⁶M, i.e. equal to or less than about 1 x 10⁻⁶ M, equal to or less than about 1 x 10⁻⁷ M, equal to or less than about 1 x 10⁻⁸ M, equal to or less than about 1 x 10⁻⁹ M, equal to or less than about 1 x 10⁻¹⁰ M, equal to or less than about 1 x 10⁻¹¹ M, or equal to or less than about 1 x 10⁻¹² M.

As mentioned above, CCR6 is also expressed on non-Th17 memory T cells and hence an antibody-drug conjugate which uses calcineurin inhibition to specifically inhibit T cell function in CCR6-expressing cells will in theory suppress the wider memory pool of T cells including, but not limited to, Th17 cells. The applicability of this invention therefore extends beyond steroid resistance to all T-cell mediated diseases, including but not limited to multiple sclerosis, rheumatoid arthritis, inflammatory bowel disease, psoriasis and asthma.

Thus, the application provides a conjugate comprising an anti-CCR6 antibody and a calcineurin inhibitor, such as tacrolimus, cyclosporin A or voclosporin, for use in a method for the treatment of T-cell mediated inflammatory disease, such as multiple sclerosis, rheumatoid arthritis, inflammatory bowel disease, psoriasis and asthma.

The conjugate for use in the invention comprises a calcineurin inhibitor linked to the anti-CCR6 antibody.

The calcineurin inhibitor may be any compound that inhibits or reduces the activity of calcineurin. Such inhibitors have been shown by the present inventors to target steroid resistant T cells. Calcineurin is also known as protein phosphatase 3 and calcium dependent serine threonine phosphatase.

Any suitable calcineurin inhibitor may be used in the conjugate described herein. Examples of preferred calcineurin inhibitors include tacrolimus, cyclosporin A (CsA), or voclosporin, or a therapeutically active fragment, derivative or mimetic thereof. Reference to a particular compound herein includes all isomeric forms, including (wholly or partially) racemic mixtures and other mixtures thereof. Cyclosporin A is also known as cyclosporine, cyclosporine, ciclosporin, Sandimmune®, Gengraf®, Neoral®, Restasis® and Sangcya®. Cyclosporin A has accession number DB00091 on the Drug Bank database (http://www.drugbank.ca/drugs/DB00091. The structure of CsA is shown in Figure 18. Most preferably, the calcineurin inhibitor is CsA, or a therapeutically active fragment or mimetic thereof.

In the method of treating an inflammatory disease disclosed herein, a therapeutically effective amount of the conjugate is administered to the subject, i.e. the conjugate is administered in an amount that has the desired therapeutic effect, e.g. by reducing or ameliorating one or more symptoms of the inflammatory disease.

In the conjugate for use in the invention, the anti-CCR6 antibody can be conjugated directly to the calcineurin inhibitor or via a linker. The linker may be any suitable linker that is known in the art. Suitable linkers include, for example, cleavable and non-cleavable linkers. A cleavable linker is typically susceptible to cleavage under intracellular conditions. Suitable cleavable linkers include, for example, a peptide linker cleavable by an intracellular protease, such as lysosomal protease or an endosomal protease. In exemplary embodiments, the linker can be a dipeptide linker, such as a valine-citrulline (val-cit) or a phenylalanine-lysine (phe-lys) linker. Other suitable linkers include linkers hydrolyzable at a pH of less than 5.5, such as a hydrazone linker. Additional suitable cleavable linkers include disulfide linkers.

Techniques for conjugating therapeutic agents to proteins, and in particular to antibodies, are well-known. (*See, e.g.,* Arnon et al., "Monoclonal Antibodies For Immunotargeting Of Drugs In Cancer Therapy," in Monoclonal Antibodies And Cancer Therapy (Reisfeld et al. eds., Alan R. Liss, Inc., 1985); Hellstrom et al., "Antibodies For Drug Delivery," in Controlled Drug Delivery (Robinson et al. eds., Marcel Dekker, Inc., 2nd ed. 1987); Thorpe, "Antibody Carriers Of Cytotoxic Agents In Cancer Therapy: A Review," in Monoclonal Antibodies '84: Biological And Clinical Applications (Pinchera et al. eds., 1985); "Analysis, Results, and Future Prospective of the Therapeutic Use of Radiolabeled Antibody In Cancer Therapy," in Monoclonal Antibodies For Cancer Detection And Therapy (Baldwin et al. eds., Academic Press, 1985); and Thorpe et al., 1982, Immunol. Rev. 62:119-58. *See also, e.g.,* PCT publication WO 89/12624.)

Typically, when using an antibody conjugated to a therapeutic agent (*i.e*., a calcineurin inhibitor in this case), the agent is preferentially active when internalized by cells to be treated.

Typically, the conjugate comprises a linker region between the calcineurin inhibitor and the antibody. As noted above, in typical embodiments, the linker is cleavable under intracellular conditions, such that cleavage of the linker releases the calcineurin inhibitor from the antibody in the intracellular environment. This allows the calcineurin inhibitor to be released once it has reached its target location.

For example, in some embodiments, the linker is cleavable by a cleaving agent that is present in the intracellular environment (*e.g*., within a lysosome or endosome or caveolea). The linker can be, *e.g.,* a peptidyllinker that is cleaved by an intracellular peptidase or protease enzyme, including, but not limited to, a lysosomal or endosomal protease. Typically, the peptidyllinker is at least two amino acids long or at least three amino acids long.

Cleaving agents can include cathepsins B and D and plasmin, all of which are known to hydrolyze dipeptide drug derivatives resulting in the release of active drug inside target cells (*see, e.g.,* Dubowchik and Walker, 1999, Pharm. Therapeutics 83:67-123). Most typical are peptidyllinkers that are cleavable by enzymes that are present in CD70-expressing cells. For example, a peptidyllinker that is cleavable by the thiol-dependent protease cathepsin-B, which is highly expressed in cancerous tissue, can be used (*e.g.*, a Phe-Leu or a Gly-Phe-Leu-Gly linker). Other such linkers are described, *e.g*., in U.S. Patent No. 6,214,345. In specific embodiments, the peptidyllinker cleavable by an intracellular protease is a Val-Cit linker or a Phe-Lys linker (*see, e.g.,* U.S. patent 6,214,345, which describes the synthesis of doxorubicin with the val-cit linker).
One advantage of using intracellular proteolytic release of the calcineurin inhibitor is that the inhibitor is typically attenuated when conjugated and the serum stabilities of the conjugates are typically high.

In other embodiments, the cleavable linker is pH-sensitive, *i.e*., sensitive to hydrolysis at certain pH values. Typically, the pH - sensitive linker is hydrolysable under acidic conditions. For example, an acid-labile linker that is hydrolysable in the lysosome (*e.g*., a hydrazone, semicarbazone, thiosemicarbazone, cis-aconitic amide, orthoester, acetal, ketal, or the like) can be used. (*See, e.g*., U.S. Patent Nos. 5,122,368; 5,824,805; 5,622,929; Dubowchik and Walker, 1999, Pharm. Therapeutics 83:67-123; Neville et al., 20 1989, Biol. Chem. 264:14653-14661.) Such linkers are relatively stable under neutral pH conditions, such as those in the blood, but are unstable at below pH 5.5 or 5.0, the approximate pH of the lysosome. In certain embodiments, the hydrolyzable linker is a thioether linker (such as, *e.g*., a thioether attached to the therapeutic agent via an acylhyclrazone bond (*see, e.g.,* U.S. Patent No. 5,622,929)).

In yet other embodiments, the linker is cleavable under reducing conditions (*e.g*., a disulfide linker). A variety of disulfide linkers are known in the art, including, for example, those that can be formed using SATA (N-succinimidyl-S-acetylthioacetate), SPDP (N-succinimidyl-3-(2-pyridyldithio)propionate), SPDI3 (N-succinimidyl-3-(2-pyridyldithio)butyrate) and SMPT (N-succinimidyl-oxycarbonyl-alpha-methyl-alpha-(2-pyridyl-dithio)toluene), SPDB and SMPT (*See, e.g*., Thorpe et al., 1987, Cancer Res. 47:5924-5931; Wawrzynczak et al., Inlmmunoconjugates: Antibody Conjugates in Radioimagery and Therapy of Cancer (C. W. Vogel ed., Oxford U. Press, 1987. *See also* U.S. Patent No. 4,880,935.)

In yet other specific embodiments, the linker is a malonate linker (Johnson et al., 1995, Anticancer Res. 15:1387-93), a maleimidobenzoyllinker (Lau et al., 1995, BioorgMed-Chem. 3(10):1299-1304), or a 3'-N-amide analog (Lau et al., 1995, Bioorg-MedChem. 3(10):1305-12).

Typically, the linker is not substantially sensitive to the extracellular environment. As used herein, "not substantially sensitive to the extracellular environment, " in the context of a linker, means that no more than about 20%, typically no more than about 15%, more typically no more than about 10%, and even more typically no more than about 5%, no more than about 3%, or no more than about 1% of the linkers, in a sample of conjugate, are cleaved when the conjugate is present in an extracellular environment (*e.g*., in plasma). Whether a linker is not substantially sensitive to the extracellular environment can be determined, for example, by incubating independently with plasma both (a) the conjugate (the "conjugate sample") and (b) an equal molar amount of unconjugated antibody or calcineurin inhibitor (the "control sample") for a predetermined time period (*e.g*., 2, 4, 8, 16, or 24 hours) and then comparing the amount of unconjugated antibody or calcineurin inhibitor present in the conjugate sample with that present in control sample, as measured, for example, by high performance liquid chromatography.

In other, non-mutually exclusive embodiments, the linker promotes cellular internalization. In certain embodiments, the linker promotes cellular internalization when conjugated to the calcineurin inhibitor. In yet other embodiments, the linker promotes cellular internalization when conjugated to both the calcineurin inhibitor and the antibody.

A variety of linkers that can be used with the present conjugates are described in WO 2004/010957 entitled "Drug Conjugates and Their Use for Treating Cancer, An Autoimmune Disease or an Infectious Disease".

When the calcineurin inhibitor is CsA, the antibody is preferably attached to the beta-hydroxyl group of the butenyl-methyl-L-threonine residue of CsA via the linker (see Figure 18). This hydroxyl group is a natural nucleophilic site for site-specific conjugation.

In a preferred embodiment, the conjugate for use in the invention comprises an anti-CCR6 antibody linked to tacrolimus or CsA.

"Treatment" of an inflammatory disease as used herein means that the conjugate has a therapeutic effect on the disease. Preferably, administration of the conjugate results in a reduction or amelioration of disease symptoms in the subject being treated. Prophylactic treatment is also included.

Inflammatory diseases are mediated by T cells and include autoimmune diseases. Non-limiting examples of inflammatory diseases to be treated in accordance with the invention include uveitis, Behçet's disease, Vogt-Koyanagi-Harada (VKH) disease, multiple sclerosis, neuromyelitis optica, rheumatoid arthritis, psoriasis, inflammatory bowel disease (including Crohn's disease and ulcerative colitis), systemic lupus erythematosus, seronegative spondyloarthropies, Sjogren's syndrome, and severe asthma. The subject may also be post-organ transplantation. Preferably, the inflammatory disease to be treated is uveitis.

Steroid resistance is a major problem when treating inflammatory disease and the present inventors have shown that the steroid resistant phenotype is associated with markers of Th17 cells. In view of this, the inflammatory disease to be treated using the conjugate described herein may be steroid resistant. In other words, the subject to be treated with the conjugate is preferably refractory to treatment with steroids. In some embodiments, the subject to be treated has been identified as steroid resistant or refractory to treatment with steroids prior to treatment with the conjugate.

The conjugate may be administered to the subject to be treated by any suitable route of administration. For example, the conjugate may be administered orally, intravenously, enterally, subcutaneously, intramuscularly, or intraperitoneally to said subject.

Preferably, the conjugate is formulated into a pharmaceutical composition comprising a pharmaceutically acceptable carrier, diluent, or excipient prior to administration to the subject. Therefore, the invention also provides a pharmaceutical composition comprising a conjugate comprising (i) an anti-CCR6 antibody and (ii) a calcineurin inhibitor, as described in detail above, for use in a method for the treatment of an inflammatory disease.

The subject to be treated or used in any of the methods disclosed herein is preferably a mammal and more preferably a human.

Prior to treatment with the conjugate described herein, expression of IL-17 and/or CCR6 may be determined by in a sample of T cells obtained from the subject to be treated. If IL-17 and/or CCR6 expression is detected at a higher level than in a reference sample of T cells or at a higher level than a reference value (e.g. amount or concentration) of IL-17 and/or CCR6 in the sample of T cells obtained from the subject, then the subject is selected for treatment with the conjugate described herein. The reference sample of T cells is preferably obtained from a normal, healthy subject.

The invention also provides the conjugate described herein for use in a method for the treatment of an inflammatory disease in a subject in need thereof, wherein expression of IL-17 and/or CCR6 in a sample of T cells obtained from the subject has been determined prior to treatment with the conjugate, such that the subject is selected for treatment if expression of IL-17 and/or CCR6 has been detected in the sample obtained from the subject at a higher level than in a reference sample of T cells or at a higher level than a reference value (e.g. amount or concentration) of IL-17 and/or CCR6. The method of treatment may include the step of determining expression of IL-17 and/or CCR6 in the sample of T cells. The sample of T cells obtained from the subject may, for example, be a venous blood sample. The reference sample of T cells is preferably obtained from a normal, healthy subject.

Expression of IL-17 and/or CCR6 may be determined at the nucleic acid (e.g. mRNA) level or the protein level by any suitable method known in the art. If IL-17 and/or CCR6 expression is detected in the sample of T cells at a higher level than in a reference sample of T cells or at a higher level than a reference value (e.g. amount or concentration) of IL-17 and/or CCR6, then the subject is selected for treatment with the conjugate described herein. In some embodiments, expression of IL-17 and/or CCR6 in the sample of T cells obtained from the subject (i.e. a test sample) may be compared to a normal reference sample of T cells obtained from a subject not suffering from an inflammatory disease and if expression of IL-17 and/or CCR6 in the test sample is greater than expression of IL-17 and/or CCR6 in the normal reference sample, then the subject is selected for treatment with the conjugate described herein.

The invention also relates to a method for identifying a subject likely to be resistant to steroid treatment. The method comprises the step of determining expression of CCR6 in a sample of T cells obtained from the subject, as described above, such that expression of CCR6 in the sample at a higher level than in a reference sample of T cells or at a higher level than a reference value (e.g. amount or concentration) of CCR6 indicates that the subject is likely to be resistant to steroid treatment. The method may also include the step of obtaining the sample of T cells from the subject. The reference sample of T cells is preferably obtained from a normal, healthy subject.

The subject has preferably been diagnosed with an inflammatory disease, such as an autoimmune disease. Examples of inflammatory diseases that may be diagnosed according to the present invention include uveitis, Behçet's disease, Vogt-Koyanagi-Harada (VKH) disease, multiple sclerosis, neuromyelitis optica, rheumatoid arthritis, psoriasis, inflammatory bowel disease (including Crohn's disease and ulcerative colitis), systemic lupus erythematosus, seronegative spondyloarthropies, Sjogren's syndrome, and severe asthma. Alternatively, the subject may be post-organ transplantation.

The invention also relates to a method for identifying a subject likely to benefit from or to respond well to treatment with a calcineurin inhibitor. The method comprises the step of determining expression of CCR6 in a sample of T cells obtained from the subject, as described above, such that expression of CCR6 in the sample at a higher level than in a reference sample of T cells or at a higher level than a reference value (e.g. amount or concentration) of CCR6 indicates that the subject is likely to benefit from or respond well to treatment with the calcineurin inhibitor. The method may also include the step of obtaining the sample of T cells from the subject. The reference sample of T cells is preferably obtained from a normal, healthy subject.

The calcineurin inhibitor is preferably targeted to Th17 cells, and is preferably for use as a conjugate, as described herein. Therefore, the invention also relates to a method for identifying a subject likely to benefit from or to respond well to treatment with the conjugate comprising (i) an antibody that binds to a Th17 cell surface marker and (ii) a calcineurin inhibitor, as described herein.

Preferably, the subject has previously been diagnosed with an inflammatory disease, such as an autoimmune disease, and more preferably with a steroid-resistant inflammatory disease. Non-limiting examples of such inflammatory diseases include uveitis, inflammatory nephritis, multiple sclerosis, rheumatoid arthritis or inflammatory bowel disease. The subject may also be post-organ transplantation. In a preferred embodiment, the subject has previously been diagnosed with uveitis.

### EXAMPLES

The inventors have shown that patients with steroid refractory intra-ocular inflammation (uveitis) have Th17 cells that are insensitive to steroid inhibition (steroid refractory; "SR"), yet susceptible to calcineurin inhibition. This same population has also been identified in steroid refractory patients suffering from inflammatory nephritis, a disease of the kidneys. The inventors have also demonstrated that the steroid refractory population of Th17 cells can be defined and targeted by the cell-surface marker CCR6.

In this study, we have used the human intraocular inflammatory disease, uveitis, to model the dynamics of steroid responses, and have demonstrated that *in vitro* Th17 cells are resistant to glucocorticoid suppression at the proliferation, cytokine protein and gene expression level and we have also validated our findings in the murine EAU model. Furthermore, we demonstrate, in both man and mouse, that Th17 cells are preferentially sensitive to the calcineurin inhibitor, CsA, thus revealing the mechanism by which calcineurin inhibitors attenuate SR diseases and highlighting the potential for Th17 cells to be exploited as a biomarker for SR disease that can be targeted with calcineurin inhibition.
**Example 1** - **Human Thl7 Cells are Resistant to Glucocorticoids.**
   Previous reports have demonstrated that Th17 cytokines are associated with a decreased PBMC response to glucocorticoids (22) and that *in vitro* murine polarized CD4+Th17 T cells are non-responsive to glucocorticoids (21). Therefore, to determine whether *in vitro,* human CD4+Th17 T cells were responsive to glucocorticoids, we generated an IL-17 secreting CD4+ T cell subset using the innate expression of CCR6. CD4⁺CCR6⁺ T cells (Th17) were activated and cultured with specific Th17 polarizing cytokines for 7 days and CD4⁺CCR6⁻ T cells (Th0) were used as a non-Th17 control (less than 1.5% IL-17 production, Fig. 5). Based on our previously published reports (19, 30), we measured the steroid response by the percentage of proliferation inhibition in the presence of the synthetic glucocorticoid, dexamethasone (Dex). Both Th17 and Th0 were treated with Dex for 24 hours and proliferation was quantified by tritiated thymidine incorporation. Proliferation of Th17 cells persisted on exposure to Dex at a concentration which effectively suppressed Th0 cell division (Fig.1A). Importantly, protein expression analysis showed that IL-17 was maintained in Dex-treated Th17 cells (Fig. 1B and 1C). Therefore, our results indicate that polarized, human CD4+Th17 T cells are non-responsive to inhibition by glucocorticoids at both the cell proliferation and IL-17 expression level.
   As a result of glucocorticoid binding the glucocorticoid receptor (GR), the GR translocates to the nucleus and binds to glucocorticoid-response elements within the DNA. In order to interrogate potential molecular mechanisms underlying the non-responsiveness of Th17 cells, we first examined the nuclear translocation of the GR in Th17 cells using confocal microscopy. Fluorescent staining showed that the GR was primarily cytoplasmic before treatment with Dex, and following 30 minutes Dex treatment, the GR translocated to the nucleus (as seen by the co-localisation of red and blue in the images) (Fig. 1D). A GR antagonist, RU486, was used as a positive control for GR translocation (data not shown)(31). We then compared the translocation of the GR in Th17 and Th0 cells. However, we did not find any difference in the nuclear density of GR between Th17 and Th0 cells after Dex treatment, indicating that GR translocation is not significantly perturbed in Th17 cells (Fig. 1E). As overexpression of the functionally inactive GR-β could be responsible for glucocorticoid resistance (32, 33), we interrogated the expression of total GR and GR-β in human Th17 cells. There was no significant difference in the expression of GR or GR-β between *in vitro* derived Th17 and Th0 cells, and this was also not altered by the addition of Dex (Fig. 1F and 1G). These results suggest that the glucocorticoid non-responsiveness we observed in Th17 cells in terms of cell proliferation and cytokine expression is not related to differences in total GR expression, GR translocation or overexpression of the dominant, negative GR-β isoform.
**Example 2** - **Genome-wide Analysis Reveals a Restricted Response by Human Thl7 Cells to Glucocorticoids.**
   In order to fully characterize the effect of glucocorticoids on Th17 and Th0 cells, we performed gene expression profiling analyses using Affymetrix U133 2.0 GeneChips. As previously carried out, Th17 and Th0 cells were cultured with Dex for 24 hours and then harvested for analysis. A total of 24 samples were analysed (6 matched sets of polarized cells each with and without Dex) Principal Component Analysis (PCA) demonstrated that the genome wide expression change induced by Dex treatment in Th17 cells was significantly restricted compared to the Dex response in Th0 cells (Fig 2A). Similarly, the total number of genes responding to Dex (defined as a 2-fold change with a p-value of <0.05) in Th17 cells was much less than in Th0 cells(Fig. 2B) The majority of genes (36) were differentially expressed only in the Th0 subset following Dex treatment. Moreover, hierarchical clustering analysis indicated that many Dex-induced gene expression changes were different between the two cell subsets (Fig. 2C). In particular, the expression of Nuclear Factor of Kappa Light Polypeptide Gene Enhancer in B-cells Inhibitor, zeta (NFKBIZ), which promotes IL17 expression (34), was suppressed in Th0 cells while its expression was increased in Th17 cells (see Table 1). Taken together, the genome-wide expression profiles provide further evidence for a human glucocorticoid resistant Th17 phenotype.
**Example 3** - **Murine Th17 Cells are Resistant to Dex but Susceptible to Calcineurin Inhibition.**
   To confirm *in vivo* that Th17 cells are unresponsive to treatment with glucocorticoids, our next step was to determine whether Th17 cell driven inflammation was SR and further, to test whether SR Th17 cells could be overcome using calcineurin inhibition. Therefore, we employed the murine platform, Experimental Autoimmune Uveitis (EAU) to investigate this. We first tested that our *in vitro* observation of continued Th17 cell proliferation in the presence of Dex was replicated in polarized murine CD4⁺ T cells expressing two types of specific T-cell receptors (OT-II and HEL-Tg) and consistent with our human data, murine Th17 cells derived from both transgenic mice were resistant to glucocorticoid suppression of proliferation. However, with treatment of the cell subsets with the calcineurin inhibitor, Cyclosporine A (CsA), the opposite response was observed. Th17 cells were fully suppressed at a drug dose that was unable to inhibit Th1 proliferation (Fig 3A).
   Next, we further tested whether calcineurin inhibition could control inflammation *in vivo,* using EAU as a model of Th17/Th1 driven autoimmunity. In order to compare the effect of Dex and CsA on disease, we need to achieve equivalent suppression of disease which was revealed following a dose titration of each drug (Fig. 5). Then, we compared the effect of Dex and CsA on suppression of clinical disease by Topical Endoscopic Fundus Imaging (TEFI; Fig. 3B and 3C). This demonstrated a significant reduction of disease severity following the first and subsequent treatments, which was also confirmed by tissue histology (Fig. 3D and 3E). Furthermore, as previously reported (35), the tissue infiltrating cells were quantified and characterized. Results showed a significant and equal reduction of CD4⁺ T cells in both Dex and CsA treated mice (Fig. 3F). The proportion of infiltrated CD11b⁺ myeloid cells was also similarly reduced in both treatment groups while Ly6G⁺ neutrophils remained unchanged from control mice and (Fig. 7A and 8B). However, despite achieving equivalent suppression of T helper cell numbers with both drugs, the IL-17 and IFN-γ cytokine profiles of these tissue infiltrating CD4⁺ cells was strikingly different. Dex effectively suppressed IFN-γ expression by 45%, while IL-17 expression was increased (Fig. 3G) in these eye infiltrating cells. In contrast, CsA almost completely ablated IL-17 expression with a 91% reduction from control mice but also significantly reduced IFN-γ expression (Fig. 3G). This drug cytokine skewing is particularly evident when the ratio of IL-17/IFN-y is calculated; Dex preferentially decreased IFN-γ while CsA, which decreased both cytokines, preferentially inhibits IL-17 expression (Fig. 3H). Consistent with this, mRNA analysis of tissue infiltrating CD4+ T cells from Dex treated mice showed that Th17 cytokines (IL-17A, IL-17F and IL-22) were significantly increased compared to control (untreated) animals (Fig. 3I). Moreover, only CsA treatment significantly reduced the expression of Th17 and Th1 specific transcription factors, *RORC, Tbx21,* and Ahr (Fig. 3I). Therefore, our data demonstrate that calcineurin inhibition can control both Th17 and Th1 mediated intraocular inflammation in EAU, partially by suppressing the expression of key transcription factors *Rorc* and *Tbx21* in the retina infiltrating CD4⁺ T cells.
**Example 4** - **Human SR Th17 cells are preferentially suppressed by the Calcineurin Inhibitor, CsA.**
   To investigate whether the glucocorticoid unresponsive Th17 cells seen in our human, polarized Th17 cultures were suppressible, we carried out experiments using the calcineurin inhibitor, CsA, to determine whether the dominant anti-Th17 effects seen in our murine work was recapitulated. Following our previous protocol, CD4⁺CCR6⁺ T cells were polarized in Th17 cytokines and CD4⁺CCR6⁻Th0 T cells were left unpolarised; both were then treated with CsA for 24 hours. Flow cytometric analysis of the protein expression demonstrated suppression of both IL-17 and IFN-γ in both Th17 and Th0 (Fig. 4A and 4B).However, as seen in the murine data, IL-17 expression was significantly more suppressed by CsA than IFN-γ (n=20; p<0.0001) (Fig. 4C). Furthermore, this magnitude of suppression became more apparent when the IL-17/IFN-γ ratio was calculated. Compared to the untreated cultures (Control), CsA treatment demonstrated preferential suppression of IL-17 protein expression compared to the Dex treated cultures (n=20; p=0.0031) (Fig. 3D). This suppression of Th17 cells by calcineurin inhibition was further reflected by reduced expression of the key Th17 transcription factor *RORC,* with concomitant ablation of *IL17A* and over 90% reduction in *IL17F* mRNA expression. This was despite continued expression of *Ahr* as well as *IL-22.* Conversely, the expression of *IFN-γ* was only reduced by 43%, and expression of the key transcription factor for Th1 differentiation (36), *Tbx21* was not changed (Fig. 4E).
   We also carried out genome wide profiling using Affymetrix U133 2.0 GeneChips to determine whether this preferential Th17 protein expression suppression was also seen at the gene level. PCA of polarized Th17 compared with Th0 cells demonstrated significant changes in gene expression were induced in Th17 cells following CsA treatment but Th0 cells only demonstrated a small expression change (Fig. 4F).
   These results demonstrate that human CD4⁺Th17 cells are preferentially suppressed by calcineurin inhibition.
**Example 5** - **CD4+ cells from patients with SR uveitis are characterised by the expression of IL-17.**
   Experimental models and *ex-vivo* human studies have definitively demonstrated that uveitis is CD4+ T-cell driven and, as in other autoimmune conditions, Th17 cells are central to in the pathogenesis of disease [15, 16].
   We have shown that patients with SR uveitis and ulcerative colitis have a corollary SR subpopulation of T-helper cells in their peripheral blood [17, 18], and subsequently demonstrated that these SR CD4+ cells are characterized by the expression of IL-17 (an established marker of the Th17 cell type). Figure 9 shows IL-17 Expression by CD4+ cells from steroid sensitive (SS) and SR uveitis patients' peripheral blood following anti-CD3 / anti-CD28 T cell receptor ligation.
**Example 6 - Selection of a Thl7 cell surface marker to target CsA to Thl7 cells.**
   A key challenge, however, is selection of a Th17 cell surface protein for CsA-monoclonal antibody (mAb) ligation. To try to resolve this we have taken a genome-wide approach, using gene expression profiling of human and murine Th17 versus unpolarised Th0 cells, to identify candidate mAb receptors. The advantage of this strategy being that no *a priori* assumptions are made about the transcripts tested.
   The scatter plots shown in Figure 10 represent the averaged microarray analyses of 4 murine CD4+ cell cultures (2x Th17 and 2x Th0) and 8 human CD4+ cell cultures (4x Th17 and 4x Th0). In the murine experiments, naive CD4+ cells were either activated in the presence of cytokines, which induce a Th17 cell phenotype, or activated and not polarized (Th0), and of the 45,101 murine probes used, CCR6 is the highest ranking cell surface protein to be expressed. It also lies directly adjacent to IL-17A, suggesting that both transcripts are closely associated. Human peripheral blood CD4+ cells were then FACS sorted on the basis of CCR6 expression and cultured for 2 weeks in the presence or absence of Th17 polarising cytokines (see below for protocol). Out of the 54,675 human probes used, CCR6 maintained its place as the top-ranked cell surface protein, and again this was clustered with Th17 cell associated cytokines and transcription factors.
   We then tested whether CCR6's association with the Th17 cell phenotype was maintained in CD4+ cell cultures from human peripheral blood (see the FACS plots in Figure 11).
   This demonstrated that CCR6- cells have minimal potential to become Th17 cells even under culture conditions which drive them toward a Th17 phenotype, and CCR6+ cells from the same individual (regardless of steroid responsiveness) default to IL-17 expression even with just TCR stimulation in the absence of polarising cytokines (Th0).
   CCR6 is therefore the strongest candidate cell surface protein for drug delivery to Th17 cells. However, CD25 is another cell surface receptor found on human memory T cells with the capacity to generate IL-17, and this also has potential to be exploited for drug delivery as it internalises its cognate ligand. As a drug target, CD25 is nonetheless weaker, as it is expressed on FoxP3+ murine T regulatory (Treg) cells. This inconsistency between mouse and man therefore makes CD25 unsuitable for rodent proof-of-concept studies.
   It is nonetheless important to highlight that humanized anti-CD25 mAbs are licensed for use in the prevention of allograft rejection in man [20] and there are also Phase II data supporting their use in uveitis [21]. Hence, humanized anti-CD25 mAbs which have already been clinically validated, and therefore would have reduced development costs and time scales, remain an alternative antibody conjugate for further evaluation following proof-of-concept in murine models of uveitis with CCR6 mediated calcineurin inhibition.
**Example 7** - **Demonstration that Th17 cells resist corticosteroid suppression compared with Th1 cells in both murine and human in vitro systems, but are susceptible to calcineurin inhibition.**
   Having shown that CD4+ cells from SR uveitis patients are biased to Th17 differentiation following T cell receptor ligation (see above), we tested the hypothesis that Th17 cells resist corticosteroid suppression compared with Th1 cells in both murine and human in vitro systems.
   Murine OT-II and HEL-Tg CD4+ cells were polarised to Th1 and Th17 and exposed to the synthetic corticosteroid Dexamethasone (Dex), or CsA. Proliferation in the polarised Th1 and Th17 cells was then quantified by tritiated thymidine incorporation, and the percentage inhibition by Dex was calculated by comparison with uninhibited controls, confirming that Th17 cells are steroid refractory. Conversely, Th17 cells were exquisitely susceptible to CsA suppression (OT-II example shown, but the same result was repeated using HEL-Tg CD4+ cells) (see Figure 12).
   In order to establish whether the same Th17 steroid refractory / CsA sensitive paradigm was replicated in human lymphocytes, we generated Th17 cells, and unpolarised (Th0) cells from human peripheral blood. Representative post-culture expression of the marker Th1 cytokine (IFNγ) and Th17 cytokine (IL-17) in CD4+ cells from healthy human donors was as shown in the following FACS dot blots shown in Figure 13A.
   IL-17 expression was not suppressed by Dex, but almost completely ablated by CsA, consistent with a SR Th17 phenotype as shown by the plots in Figure 13B.
   The ratio of IL-17 to IFN-γ also reduced by 47.9% after cyclosporine treatment, demonstrating that IL-17 is preferentially inhibited relative to IFN-γ (n=10 healthy volunteers) (see Figure 14).
   Extending this to our disease cohort, we applied a genome-wide approach to interrogate the gene expression profiles of Th17 and Th0 cells from SR (n=3) and SS (n=3) uveitis patients, before and after Dex (D) treatment (total = 24 microarrays). Principal component analysis of the data presented in the above heat map confirms a reduced genome-wide response to Dex in Th17 cells from both SS and SR individuals (data not shown).
**Example 8** - **Internalisation of anti-CCR6 antibodies by human CD4+ cells.**
   We have demonstrated that anti-CCR6 mAbs are internalised on receptor ligation in human CD4+ cells, validating this as a target for drug delivery (see Figure 15). This was investigated using three commercially available human anti-CCR6 antibodies.
**Example 9 - Proof of principle using a mouse model of autoimmune uveitis.**
   Proof of principle data using the model disease experimental autoimmune uveitis in B10RIII mice showed that Il-17 expression is maintained (and even increased) at the site of inflammation in the retina following Dex treatment, whereas intracellular cytokine expression is unchanged in splenic CD4⁺ T cells (see Figure 16). In contrast, CsA completely suppressed IL-17 expression in retina-infiltrating CD4+ cells (see Figure 17).
**Example 10** - **Internalisation of CCR6 in human CD4+ cells in peripheral blood mononuclear cell (PBMC) culture and isolated CD4+ T cells.** Figure 19 shows that CCR6 is internalised equally in human CD4+ cells in peripheral blood mononuclear cell (PBMC) culture and isolated CD4+ T cells. Potentially, CCR6:anti-CCR6 mAb complexes could be removed from the surface of CD4+ T cells by receptors on the surface of other cells within the PBMC population. However, internalisation is similar on both CD4+ T cell in a mixed PBMC population to that in isolated CD4+ T cell, indicating that this is unlikely. (A reduction in % CCR6 expression at 1 hr relative to from the control sample (ice) indicates that the CCR6:mAb complex has become internalised.)
   The data shown in Figure 20 provide confirmation that anti-CCR6 mAb internalises on human CD4+ T cells. The proportion of quenching inversely corresponds to the proportion of internalised CCR6 and the reduction in quenching from the control sample (ice) after 1 hour indicated that the CCR6:mAb complex has become internalised.
   Therefore, anti-CCR6 monoclonal antibodies are internalised in human CD4+ T cells, and this is not reduced in mixed PBMC cultures, the implication being that the antibody will be able to deliver conjugated drug inside T cells in whole blood, confirming that CCR6 is a tractable ADC target.
**Example 11** - **CCR6 expression on immune cell subsets in the blood of human normal volunteers.**
   Figure 21B shows that CCR6 expression in the T cell subsets is greatest in the memory CD4+ T cell population. A high proportion of B cells also express CCR6 (see Figure 21C). However, it has previously been shown that calcineurin inhibitors do not directly affect B cell function (65).
   Therefore, CCR6 is predominantly expressed on the intended target population of memory CD4+ T cells. CCR6 is also highly expressed on B cells, but these will not be affected by calcineurin inhibition (see Figure 21). The absolute number of CCR6 receptors on immune cells subsets in the blood of human normal volunteers is shown in Figure 22. The absolute number of CCR6 receptors is higher on memory than naive T cells, indicating that there should be a sufficient number of receptors per cell to efficiently internalise the ADC. In the non-T cell subsets, B cells again have the highest density of CCR6 receptors per cell, but this should not be biologically significant in the context of this intervention as they are not susceptible to calcineurin inhibition (see Figure 22).
**Example 12** - **Suppression of IL-17A and IFNγ cytokine production by cyclosporine A and tacrolimus in Th17 cells.**
   The data shown in Figure 23 demonstrate that the calcineurin inhibitors cyclosporine A (CsA) or tacrolimus (Tac) equally suppress IL-17A production in human Th17 (CCR6+) cells and IFNγ production in human Th0 (CCR6⁻) and Th17 cells (Fig. 23B)
   Therefore, tacrolimus has the same effect as cyclosporine A in suppressing IL-17 expression in Th17 cells and IFNγ expression in both Th17 and control (Th0) cells, confirming that this is a class effect of calcineurin inhibitors (rather than specific to cyclosporine A). The dose required to achieve this is 20x less with tacrolimus than cyclosporine A, indicating that even if the ADC delivers 20x less tacrolimus than cyclosporine A per cell, it will still be effective (Figure 23).
**Example 13** - **Internalisation of CCR6 in mouse CD4⁺ T cells.**
   Figure 24 shows that CCR6 is internalised on CD4⁺ T cells in whole splenocyte populations in B10.RIII mice. There is about 50% CCR6 internalisation after 24hr in the whole splenocyte population and on gated CD4⁺ cells (see Figure 24B).
   Murine CD4+ T cells in a mixed splenocyte population also internalise anti-CCR6 monoclonal antibodies, but this takes longer than in human cultures. Regardless, there is good internalisation in less than 24hrs (see Figure 24), which demonstrates that anti-murine CCR6 ADCs will be effective in murine *in vivo* models of inflammation, as these take weeks to develop. Hence, murine experimental systems should be appropriate for *in vivo* proof of concept studies.
**Example 14** - **Inhibition of T cell proliferation by cyclosporine A and tacrolimus.**

As shown in Figure 25A, proliferation as measured by tritiated thymidine incorporation (ccpm) was minimal following both cyclosporine A and tacrolimus treatment. When compared to the uninhibited control (Dex 10⁻¹² M), this translated to greater than 90% suppression of T cell division on average following both low and high TCR stimulation (see Figure 25B). Therefore, both calcineurin inhibitors (cyclosporine A and tacrolimus) fully inhibited CD4⁺ cell proliferation and this was achieved with a 20-fold lower concentration of tacrolimus compared with cyclosporine A.

This shows that tacrolimus at a dose of 10ng/ml achieves the same inhibition of CD4+ T cell proliferation as cyclosporine A achieves at 100ng/ml.

Hence tacrolimus is an effective inhibitor of both T cell pro-inflammatory cytokine expression and proliferation (i.e. cell division).

### DISCUSSION

Our data demonstrate *inter alia* that human and murine Th17 cells are not suppressible by glucocorticoids but are preferentially inhibited by calcineurin inhibition.

Previous reports have already suggested, in a Th2 driven murine inflammatory disease model, that Th17 cells may play an important role in perpetrating glucocorticoid resistance (21). In the current study we have showed that Th17 cells are also resistant to glucocorticoids in both human *in vitro* cultures and in a murine model of Th1/Th17 mediated autoimmunity. More importantly, we have profiled the genome wide expression pattern of glucocorticoid responses in CD4⁺ T cells and demonstrated that the overall change in the transcription program induced by glucocorticoids is restricted in human Th17 cells. This is the first report of human transcriptome-wide glucocorticoid responses in this specific type of inflammatory helper T cell. Therefore our data provide a blueprint for understanding the differential effects of glucocorticoids on these critical mediators of inflammation.

Although genetic variations, including mutations and polymorphisms of GR, contribute to congenital and generalised glucocorticoid resistance, it is more common to acquire glucocorticoid resistance from localized and disease-associated microenvironmental, and therefore epigenetic, changes as a consequence of chronic diseases (12). The glucocorticoid resistant phenotype of Th17 cells but not Th0 cells from the same patients provides strong support of the notion that such epigenetic regulation of disease-associated inflammatory Th17 cells is responsible for their insensitivity to glucocorticoid treatment.

Our genome-wide gene expression profiling data also suggest that the difference of glucocorticoid responses in helper T cells is beyond the expression of key cytokines, i.e. IFNG and IL17A. This is consistent with previous reports that the extremely diverse effects of glucocorticoid receptors in different types of cells are dramatically dependent upon the pre-existing chromatin architecture governed by different regulatory factors in different cells (38, 39). The activation of transcription regulators NF-κB and STAT3 by Th17 polarizing cytokines IL-1β, IL-G, and IL-23 has been reported to antagonize the binding of GR to their natural binding sites, therefore may serve as the molecular mechanism by which Th17 cells are resistant to the glucocorticoid suppression.

In addition, we have shown that although Th17 cells are resistant to glucocorticoids, they are selectively susceptible to calcineurin inhibition. Our data demonstrate that calcineurin inhibition suppresses the tissue infiltration and proliferation of Th17 cells, partly through attenuation of the key transcription factor RORC. Conversely, CsA did not change the expression of TBX21, the critical transcription factor for Th1 differentiation, and consequently has less effect on IFNG expression. This suggests that preferential suppression of Th17 cells by calcineurin inhibition may be central to their long-observed efficacy in the treatment of glucocorticoid resistant disease.

Furthermore, we have shown that calcineurin inhibition has a comparatively limited effect on *AHR* and *IL22* expression in Th17 cells. This suggests that calcineurin inhibition differentially targets transcription programs in Th17 cells which govern the expression of IL17A, IL17F, and IL22. This supports previous reports that AHR mediated IL22 regulation in Th17 cells is independent of the regulation of IL17A and IL17F (40, 41).

For many years, glucocorticoid resistant inflammation has been controlled by calcineurin inhibition, and our data strongly suggest that this is achieved through the preferential suppression of Th17 cells. In addition, our data provides a rationale for further evaluating the potential to selectively deliver low-dose CsA to Th17 cells to overcome glucocorticoid resistant disease.

While investigating the proliferative, protein and genome response of CCR6+Th17 cells to Dex, we could not ignore the potential contribution of the GR to SR disease. Initially, we investigated the trafficking of the GR translocation to the nucleus as a potential mechanism underlying the decreased Dex response seen in the CCR6+Th17 population and were unable to show any differences in the trafficking. This is corroborated by a study using a murine model of asthma illustrating similar trafficking in Th17 and Th2 murine cells (21). We do acknowledge that we have not investigated binding at the GRE once the GR has trafficked to the nucleus and further investigations into GR binding areas at IL-17 *loci* are also warranted. This is principally due to the surrounding literature demonstrating altered/decreased GR binding affinity in PBMCs from SR asthma patients, that can be reversed depending on the cytokine milieu (42, 43). However, the caveat inherent within this data is the suggestion that SR is a function of disease rather than a feature of the immune system that could be exacerbated during inflammation (42, 44). Nonetheless data showing inhibited NFAT binding due to GR translocation and binding at the GRE has been demonstrated in murine Th17 cells and thus it may be unlikely that affected GR binding is the cause for SR in Th17 cells (21).

### Materials and Methods

**Patients.** All protocols were approved by institutional review boards, and written informed consent was provided by all patients to the University of Bristol Eye Hospital, UK and National Institutes of Health (NIH), US.

**Mice.** B10.RIII (Harlan UK Limited, Oxford, UK), C57BL/6 OT-II (45) (Gifted from Dr. S Anderton, University of Edinburgh, UK), and 3A9 mice (46) were established and bred within the Animal Services Unit at the University of Bristol. All mice were housed in specific pathogen-free conditions with water and food available *ad libitum.* Female mice between 6 and 8 weeks were immunized for Experimental Autoimmune Uveitis (EAU) while both male and female mice were used in the Th1 and Th17 polarization experiments. All mice were kept in the animal house facilities of the University of Bristol, according to Home Office regulations. Treatment of animals conformed to the Association for Research in Vision and Ophthalmology animal policy (ARVO Statement for the Use of Reagents in Ophthalmic and Vision Research).

**Reagents.** Human RBP-3₁₆₁₋₁₈₀ peptide (SGIPYTTSYLHPGNTILHVD), hen egg lysozyme and chicken ovalbumin₃₂₃₋₃₃₉ (ISQAVHAAHAEINEAGR) were purchased from Sigma-Aldrich (US). The purity of them was determined by high performance liquid chromatography. Peptide preparations were then aliquoted and stored at -80°C. Dexamethasone (Sigma-Aldrich) (US) for cell culture was dissolved in ethanol and RPMI-1640 (PAA Laboratories Ltd, UK) to a concentration of 1x10⁻³ M. Dex for treatment of EAU was purchased from Organon Laboratories Ltd (UK). This was diluted to the appropriate concentration in phosphate buffered solution (PAA, Laboratories Ltd, UK). Cyclosporine A for cell culture and in vivo treatment of EAU mice was obtained from Sigma-Aldrich (US) and Novartis (US), respectively.

**EAU Induction and Treatment.** B10.RIII mice were immunized subcutaneously with 50µg RBP-3₁₆₁₋₁₈₀ emulsified with complete Freud's adjuvant supplemented with 1.5mg/ml *Mycobacterium tuberculosis* complete H37 Ra (BD Biosciences, US) (1:1 v/v) (47). Mice also received 1ug *Bordetella pertussis* toxin (Tocris Bioscience, US) intraperitoneally. Dex was administered subcutaneously, while CsA was administered by oral gavage.

**EAU Clinical Assessment.** The topical endoscopy fundus imaging (TEFI) was used to assess clinical score of EAU scores (48). An endoscope with a tele-otoscope, 5cm in length and 3mm outer diameter (1218AA, Karl Storz, Tuttlingen, Germany) was connected to a Nikon D80 digital camera with a 10-million pixel charge-coupled device image sensor and Nikkor AF 85/F1.8 D objective (Nikon), with an additional +4.00 diopter magnifying lens. Pupils were dilated using topical tropicamide 1% and phenylephrine 2.5% (Chauvin Pharmaceuticals Ltd, UK) and corneas were anesthetized with oxybuprocaine 0.4% (Chauvin Pharmaceuticals Ltd, UK). Images were obtained from both eyes with direct corneal contact with the endoscope. Images were processed using Photoshop CS4 software (Adobe, US). The fundal images were scored according to inflammatory changes at the optic disc and around retinal vessels, retinal lesions and structural damage (see Table 2) (49). Scores were added together for a total disease score. Disease scores that obtained over 15 on day 18 were excluded from experiments and those eyes with a disease that was too severe to have a clear view of the optic disc and retinal vessels were excluded from scoring.

**Organ Digestion.** Retinal infiltrating cells were isolated from retinal tissue using a tissue dissociation method in order to obtain a single cell suspension. Briefly, retinal tissue was dissected and chopped into small fragments and mechanically disrupted using a 25G needle and syringe (BD Bioscience, US). Tissue fragments were then forced through a 40-µm cell strainer (BD Bioscience, US) which was rinsed thoroughly with HBSS (PAA Laboratories Ltd, UK) containing 5% fetal calf serum (PAA Laboratories Ltd, UK) and the cell suspension was centrifuged to obtain a cell pellet. Cells were then resuspended in either staining buffer (balanced salt solution with 0.1% bovine serum albumin and 0.08% sodium azide) for cell surface marker staining or in RPMI-1640 medium (PAA Laboratories Ltd, UK) for stimulation with PMA and ionomycin (Sigma-Aldrich, US). Spleen tissue was dissociated into a single cell suspension using a 40-µm cell strainer. Red blood cells were lysed and cells were resuspended, as for retinal cells.

**Human Peripheral Blood CD4⁺ T cell Isolation.** CD4⁺ T cells were isolated from whole blood by negative selection. Briefly, uncoagulated blood was incubated with RosetteSep® Human CD4⁺ T cell Enrichment Cocktail (Stemcell Technologies, Canada) according to manufacturer's instructions. After a short incubation, the blood was layered over density medium (Ficoll-Paque PLUS, GE Healthcare, US) and centrifuged at 1200 x g for 25 minutes. CD4⁺ cells were removed from the interface between the plasma and density gradient and washed twice in 10% FCS containing RPMI-1640 (PAA Laboratories Ltd, UK). Routinely, the purity of CD4⁺ T cells achieved was greater than 95%.

**Cell Cultures.** Murine Naive T cells were obtained from lymph nodes and spleens of OT-II / 3A9 mice using CD4 positive selection kits following the manufacturer's instructions (Miltenyi Biotech). Splenocytes from respective wild-type mice were irradiated and mixed with the CD4⁺ T cells at a 1:10 ratio. Activation and polarization were achieved with either 2 µg/ml ovalbumin or 2 µg/ml Hen egg lyzosyme along with Th1 or Th17 polarizing cytokines, as previously reported (50). All cells were kept in a 37 °C, 5% CO₂ humidified incubator. Cells were used after two rounds of activation. Human peripheral CD4⁺CCR6⁻ (Th0) and CD4⁺CCR6⁺ (Th17) cells were FACS sorted, resuspended to 2x10⁶ cells/ml in complete RPMI-1640 (Invitrogen) supplemented with 10% FCS, L-glutamine and Penicillin/streptomycin (all PAA Laboratories Ltd, UK), stimulated with plate-bound 5 µg/ml anti-CD3 and 5 µg/ml anti-CD28 antibodies (eBioscience, US) (Th0) or with plate-bound antibodies and a polarizing cytokine cocktail of 20 ng/ml IL-6, 10 ng/ml IL-23, 10 ng/ml IL-1β (R&D Systems, US), 100 ng/ml anti-IFN-γ, 100 ng/ml anti-IL-4 (eBioscience, US) (Th17) for 3 days. Thereafter, cells were transferred to fresh plates and cultured in complete RPMI-1640 containing 50 ng/ml IL-2 only (for Th0) or 50 ng/ml IL-2 plus the polarizing cytokine cocktail (for Th17) for 4 days. This 7-day process was repeated once. Further experiments were all performed at day 14.

**T Cell Proliferation Assays.** T cell proliferation was measured by pulsing with 18.5 kBq [³H] thymidine (GE Healthcare, US) per well for the final 12 hours of cultures and determining thymidine uptake in counts per minute (c.p.m).

**Flow Cytometry and FACS Sorting.** Nonspecific antibody binding was blocked by incubating cells with 24G2 cell supernatant for 10 minutes at 4 °C followed by incubation with combinations of primary antibodies against cell surface markers at 4°C for 20 minutes. The preconjugated antibodies used for the staining of murine cells were: anti-CD4 (1:400 dilution; clone Rm4-5; BD Bioscience), anti-CD45 (1:1000 dilution; clone 30-F11; BD Bioscience), anti-CD11b (1:400; clone M1/70; BD Bioscience) and anti-Ly6G (1:100 dilution; clone 1A8; BD Bioscience). Intracellular cytokine staining was carried out by incubating cells with 20 ng/ml phorbol 12-myristate 13-acetate (PMA) and 1 µM inomycin for 4 hours at 37 °C. After stimulation, cell surface markers were stained and then cells were fixed, permeabilized (Cytofix/perm solution; BD Bioscience) and stained with anti-IL-17 (1:100 dilution; clone TC11-18H10; BD Bioscience) and anti-IFN-γ (1:100 dilution; clone XMG 1.2; BD Bioscience). Human cells were washed in wash buffer (PBS supplemented with 1% FCS) and stained with antibodies for 30 minutes at 4 °C using anti-CD4 (1:50 dilution; clone OKT-4; eBioscience), anti-CD3 (1:100 dilution; clone UCHT1; BD Bioscience), biotinylated anti-CCR6 (CD196)/ streptavidin-APC (1:200 dilution, BD Bioscience), anti-IL-17 (1:50 dilution; clone eBio63CAP17; eBioscience), and anti-IFN-γ (1:50; clone 4S.B3; eBioscience). All samples were acquired using a BD LSR II (BD Bioscience) and data analysis was run using FlowJo 7.6 software (Treestar). Human peripheral blood cells were FACS-sorted for CD4⁺CCR6⁺ and CD4⁺CCR6⁻ using BD Influx™ system (BD Bioscience) and routinely > 95% purity was achieved.

**Quantitative Real-time PCR.** Total RNA was extracted using the RNeasy mini kit (Qiagen, UK), followed by DNase treatment to remove contaminating genomic DNA using Turbo DNA Free Kit (Invitrogen, UK). RNA was reverse transcribed using the Promega Improm-II Reverse Transcriptase system (Promega, UK) and a G-storm Thermocycler (G-storm, UK). Real-time PCR was carried out using the StepOnePlus Real-Time PCR system (Applied Biosystems, US). The following Taqman Assays from Applied Biosystems were used: total GR (Hs00353740_m1), GRβ (Hs00354508_m1), GAPDH (Hs03929097_g1), 18s rRNA (Hs03928990_g1), IL17A (Hs00936345_m1), IL17F (Hs00369400_m1), IL22 (Hs01574154_m1), RORC (Hs01076122_m1), IFNG (Hs00989291_m1), TBX21 (Hs00203436_m1), AHR (Hs00169233), I117a (Mm00439618_m1), I117f (Mm00521423_m1), 1122 (Mm01226722_g1), Ifng (Mm01168134_m1), Rorc (Mm01261022_m1), Tbx21 (Mm00450960_m1), Ahr (Mm00478932_m1). **Confocal Microscopy.** To optimise the quantification of GR trafficking, freshly isolated CD4+ T cells (rested overnight in serum free media) were treated with 10⁻⁶ M dexamethasone (Sigma-Aldrich, US) dissolved in RPMI 1640 or GR antagonist 100 µM RU486 (Sigma-Aldrich, US) dissolved in DMSO for 30 minites. Cells were collected by centrifugation, fixed in 2% paraformaldehyde at room temperature for 15 minutes, and washed in cold PBS with 0.5% FCS and 2 mM EDTA. Cells at a concentration of 2x10⁶ cells/ml suspended in cold PBS with 0.5% FCS and 2 mM EDTA were cytospun on to poly-L lysine (Sigma-Aldrich, US) coated slides at 1500rpm for 5 minutes using Shandon EZ double cytofunnel (Thermo Scientific, UK). The area of interest was outlined with hydrophobic pen (H-400 Immedge, Vector Laboratories, UK) and left to dry overnight. Cells were then permeabilized with 0.1% TritonX (Sigma-Aldrich, US) and 1% human AB serum for 5 minutes, washed in PBS for 5 minutes, and stained with 1:300 anti-GR mAb (SC-56851, Santa Cruz, CA) in Triton X buffer for overnight at 4°C. Slides were washed with PBS for 5 minutes and incubated with the secondary antibody AF568 (A11004, Invitrogen, Paisley, UK) was applied at a concentration of 1:500 in Triton X buffer at room temp for 2 hours, followed by washing in PBS for 5 minutes. The cover slips were mounted in hard setting vectashield containing DAPI (H1500 Vector laboratories, UK). Nuclear translocation of GR was quantified using Leica SP5 confocal imaging system with Leica DMI 6000 inverted microscope and motorised XYZ stage for multiple-site imaging. For 3D image preparation of T cells, z-scans in 0.5µm increments were taken through the whole cell thickness using a 63x 1.4 aperture oil immersion lens with pinhole diameter set to 1.0 Airy disk unit, determined empirically to be optimal for this sample. Full depth z-stack images were acquired. The fluorescence was analyzed using Volocity 6.2 (Perkin Elmer, UK) within delineation via nuclear staining (DAPI) to facilitate quantification of red (GR) staining. The nuclear density of GR was expressed as total red divided by nucleus volume.

**Affymetrix Microarray Data Collection and Analysis.** Total RNA was isolated from cultured cells using mirVana miRNA isolation kit (Ambion, TX). 500 ng total RNA was amplified and biotin-labelled using MessageAmp II-Biotin Enhanced Kit (Ambion, TX). Approximately 10 µg of total labeled RNA was hybridized to GeneChip U133 plus 2.0 arrays (Affymetrix, CA) according to the manufacturer's protocols. Expression values were determined using GeneChip Operating Software (GCOS) v1.1.1. All data analysis was performed using GeneSpring GX 11.0 (Agilent Technologies, CA). Expression values for each probe were normalized using Robust Multichip Average (RMA) method. All probes with expression values <50 in all samples were deleted from subsequent analysis.

**Statistical Analysis.** Data are expressed as mean ± SEM and differences were analyzed by Mann-Whitney U test with significance determined as P < 0.05. All statistical analysis was performed using Prism version 6 (GraphPad Software Inc.).

**Table 1. Genes Differentially Induced by Dex in Th0 and Th17 Cells**

| **Probe set ID** | **Gene Symbol** | **Fold Change in Th0 Cells** | **Fold Change in Th17 Cells** | **Gene Title** |
|---|---|---|---|---|
| **223217_s_at** | NFKBIZ | -1.5 | 2.3 | nuclear factor of kappa light polypeptide gene enhancer in B-cells inhibitor, zeta |
| **209257_s_at** | SMC3 | -1.9 | 1.5 | structural maintenance of chromosomes 3 |
| **212079_s_at** | MLL | -1.7 | 1.6 | myeloid/lymphoid or mixed-lineage leukemia (trithorax homolog, Drosophila) |
| **221830_at** | RAP2A | -2.0 | 1.3 | RAP2A, member of RAS oncogene family |
| **209024_s_at** | SYNCRIP | -1.4 | 1.8 | synaptotagmin binding, cytoplasmic RNA interacting protein |
| **204415_at** | IFI6 | -2.0 | 1.2 | interferon, alpha-inducible protein 6 |
| **203946_s_at** | ARG2 | -2.6 | -1.1 | arginase, type II |
| **244774_at** | PHACTR2 | -1.5 | 1.5 | phosphatase and actin regulator 2 |
| **229450_at** | IFIT3 | -2.6 | -1.1 | interferon-induced protein with tetratricopeptide repeats 3 |
| **205266_at** | LIF | -1.7 | 1.3 | leukemia inhibitory factor (cholinergic differentiation factor) |
| **202269_x_at** | GBP1 | -1.8 | 1.2 | guanylate binding protein 1, interferon-inducible, 67kDa |
| **241343_at** | RNASEH1 | -1.4 | 1.6 | MRNA RNase HII |
| **202082_s_at** | SEC14L1 | -1.5 | 1.4 | SEC14-like 1 (S. cerevisiae) |
| **1554821_a_at** | ZBED1 | -1.5 | 1.4 | zinc finger, BED-type containing 1 |
| **208602_x_at** | CD6 | -1.8 | 1.2 | CD6 molecule |
| **203147_s_at** | TRIM14 | -1.5 | 1.4 | tripartite motif-containing 14 |
| **200730_s_at** | PTP4A1 | -1.6 | 1.3 | protein tyrosine phosphatase type IVA, member 1 |
| **236356_at** | NDUFS1 | -1.3 | 1.6 | NADH dehydrogenase Fe-S protein 1, 75kDa (NADH-coenzyme Q reductase) |
| **209871_s_at** | APBA2 | 1.7 | -1.2 | amyloid beta (A4) precursor protein-binding, family A, member 2 |
| **235683_at** | SESN3 | 2.2 | 1.0 | sestrin 3 |
| **228101_at** | APBA1 | 4.7 | 2.1 | amyloid beta (A4) precursor protein-binding, family A, member 1 |
| **216876_s_at** | IL17A | -1.1 | -2.6 | interleukin 17A |
| **225681_at** | CTHRC1 | 3.8 | 1.3 | collagen triple helix repeat containing 1 |
| **206942_s_at** | PMCH | 4.8 | 1.6 | pro-melanin-concentrating hormone |

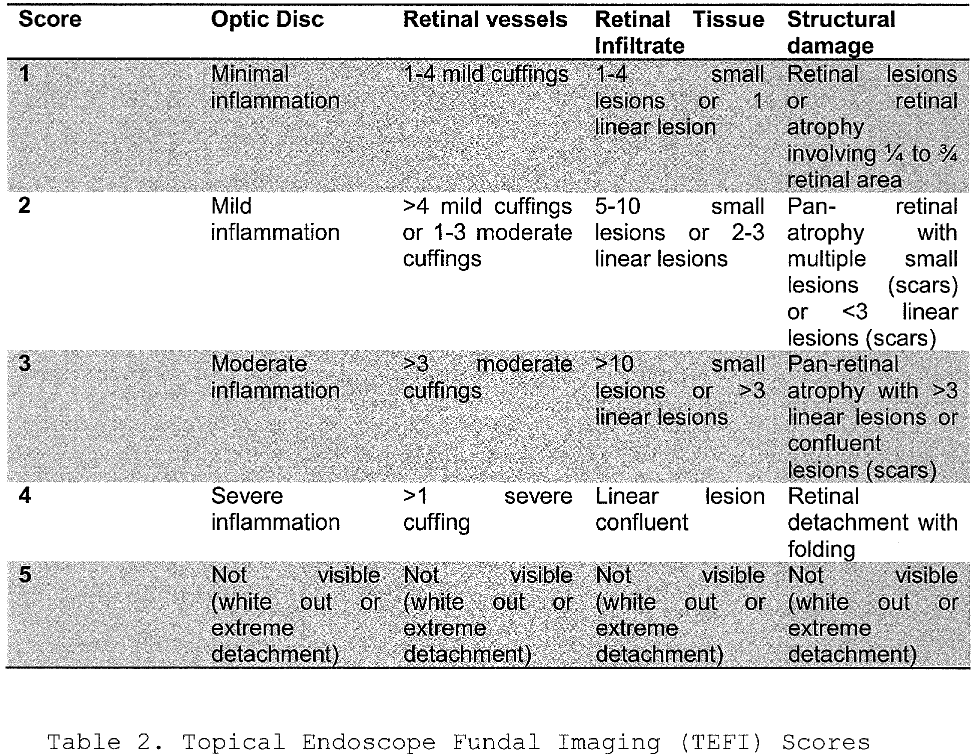

### References

1. Basu R, Hatton RD, & Weaver CT (2013) The Th17 family: flexibility follows function. Immunol Rev 252(1):89-103.
2. Cosmi L, Liotta F, Maggi E, Romagnani S, & Annunziato F (2011) Th17 cells: new players in asthma pathogenesis. Allergy 66(8):989-998.
3. Ivanov, II, et al. (2006) The orphan nuclear receptor RORgammat directs the differentiation program of proinflammatory IL-17+ T helper cells. Cell 126(6):1121-1133.
4. Wilson NJ, et al. (2007) Development, cytokine profile and function of human interleukin 17-producing helper T cells. Nat Immunol 8(9):950-957.
5. Acosta-Rodriguez EV, et al. (2007) Surface phenotype and antigenic specificity of human interleukin 17-producing T helper memory cells. Nat Immunol 8(6):639-646.
6. Singh SP, Zhang HH, Foley JF, Hedrick MN, & Farber JM (2008) Human T cells that are able to produce IL-17 express the chemokine receptor CCR6. J Immunol 180(1):214-221.
7. Annunziato F, et al. (2007) Phenotypic and functional features of human Th17 cells. J Exp Med 204(8):1849-1861.
8. Weaver CT, Elson CO, Fouser LA, & Kolls JK (2013) The Th17 pathway and inflammatory diseases of the intestines, lungs, and skin. Annual review of pathology 8:477-512.
9. Zuniga LA, Jain R, Haines C, & Cua DJ (2013) Th17 cell development: from the cradle to the grave. Immunol Rev 252 (1) :78-88.
10. Amadi-Obi A, et al. (2007) TH17 cells contribute to uveitis and scleritis and are expanded by IL-2 and inhibited by IL-27/STAT1. Nature medicine 13(6):711-718.
11. Barnes PJ & Adcock IM (2009) Glucocorticoid resistance in inflammatory diseases. Lancet 373(9678):1905-1917.
12. Yang N, Ray DW, & Matthews LC (2012) Current concepts in glucocorticoid resistance. Steroids 77(11):1041-1049.
13. Schewitz LP, Lee RW, Dayan CM, & Dick AD (2009) Glucocorticoids and the emerging importance of T cell subsets in steroid refractory diseases. Immunopharmacology and immunotoxicology 31(1):1-22.
14. Fan H & Morand EF (2012) Targeting the side effects of steroid therapy in autoimmune diseases: the role of GILZ. Discovery medicine 13(69):123-133.
15. van Rossum EF & van den Akker EL (2011) Glucocorticoid resistance. Endocrine development 20:127-136.
16. Miranda TB, Morris SA, & Hager GL (2013) Complex genomic interactions in the dynamic regulation of transcription by the glucocorticoid receptor. Molecular and cellular endocrinology*.*
17. Oakley RH, Jewell CM, Yudt MR, Bofetiado DM, & Cidlowski JA (1999) The dominant negative activity of the human glucocorticoid receptor beta isoform. Specificity and mechanisms of action. The Journal of biological chemistry 274(39):27857-27866.
18. Xystrakis E, et al. (2006) Reversing the defective induction of IL-10-secreting regulatory T cells in glucocorticoid-resistant asthma patients. The Journal of clinical investigation 116(1):146-155.
19. Lee RW, Schewitz LP, Nicholson LB, Dayan CM, & Dick AD (2009) Steroid refractory CD4+ T cells in patients with sight-threatening uveitis. Invest Ophthalmol Vis Sci 50(9):4273-4278.
20. Lee RW, et al. (2007) CD4+CD25(int) T cells in inflammatory diseases refractory to treatment with glucocorticoids. Journal of immunology (Baltimore, Md. : 1950) 179(11) :7941-7948.
21. McKinley L, et al. (2008) TH17 cells mediate steroid-resistant airway inflammation and airway hyperresponsiveness in mice. J Immunol 181(6) :4089-4097.
22. Vazquez-Tello A, Halwani R, Hamid Q, & Al-Muhsen S (2013) Glucocorticoid Receptor-Beta Up-Regulation and Steroid Resistance Induction by IL-17 and IL-23 Cytokine Stimulation in Peripheral Mononuclear Cells. Journal of clinical immunology 33(2):466-478.
23. Ano S, et al. (2013) Transcription Factors GATA-3 and RORgammat Are Important for Determining the Phenotype of Allergic Airway Inflammation in a Murine Model of Asthma. J Immunol.
24. Nanzer AM, et al. (2013) Enhanced production of IL-17A in patients with severe asthma is inhibited by 1alpha, 25-dihydroxyvitamin D3 in a glucocorticoid-independent fashion. The Journal of allergy and clinical immunology*.*
25. Nussenblatt RB, Palestine AG, & Chan CC (1983) Cyclosporin A therapy in the treatment of intraocular inflammatory disease resistant to systemic corticosteroids and cytotoxic agents. American journal of ophthalmology 96(3):275-282.
26. Chi W, et al. (2008) Upregulated IL-23 and IL-17 in Behcet patients with active uveitis. Invest Ophthalmol Vis Sci 49(7):3058-3064.
27. Chi W, et al. (2007) IL-23 promotes CD4+ T cells to produce IL-17 in Vogt-Koyanagi-Harada disease. The Journal of allergy and clinical immunology 119(5):1218-1224.
28. Kacmaz RO, et al. (2010) Cyclosporine for ocular inflammatory diseases. Ophthalmology 117(3):576-584.
29. Matsuda S & Koyasu S (2000) Mechanisms of action of cyclosporine. Immunopharmacology 47(2-3):119-125.
30. Lee RW, et al. (2007) CD4+CD25int T Cells in Inflammatory Diseases Refractory to Treatment with Glucocorticoids. J Immunol 179(11) :7941-7948.
31. Peeters BW, Ruigt GS, Craighead M, & Kitchener P (2008) Differential effects of the new glucocorticoid receptor antagonist ORG 34517 and RU486 (mifepristone) on glucocorticoid receptor nuclear translocation in the AtT20 cell line. Ann N Y Acad Sci 1148:536-541.
32. Webster JC, Oakley RH, Jewell CM, & Cidlowski JA (2001) Proinflammatory cytokines regulate human glucocorticoid receptor gene expression and lead to the accumulation of the dominant negative beta isoform: a mechanism for the generation of glucocorticoid resistance. Proceedings of the National Academy of Sciences of the United States of America 98(12):6865-6870.
33. Tissing WJ, et al. (2005) Expression of the glucocorticoid receptor and its isoforms in relation to glucocorticoid resistance in childhood acute lymphocytic leukemia. Haematologica 90(9):1279-1281.
34. Okamoto K, et al. (2010) IkappaBzeta regulates T(H)17 development by cooperating with ROR nuclear receptors. Nature 464(7293):1381-1385.
35. Kerr EC, Raveney BJ, Copland DA, Dick AD, & Nicholson LB (2008) Analysis of retinal cellular infiltrate in experimental autoimmune uveoretinitis reveals multiple regulatory cell populations. J Autoimmun 31(4):354-361.
36. Szabo SJ, et al. (2000) A novel transcription factor, T-bet, directs Th1 lineage commitment. Cell 100(6):655-669.
37. Vitale AT, Rodriguez A, & Foster CS (1996) Low-dose cyclosporin A therapy in treating chronic, noninfectious uveitis. Ophthalmology 103(3) :365-373; discussion 373-364.
38. John S, et al. (2011) Chromatin accessibility pre-determines glucocorticoid receptor binding patterns. Nature genetics 43(3):264-268.
39. Uhlenhaut NH, et al. (2013) Insights into negative regulation by the glucocorticoid receptor from genome-wide profiling of inflammatory cistromes. Molecular cell 49(1):158-171.
40. Qiu J, et al. (2012) The aryl hydrocarbon receptor regulates gut immunity through modulation of innate lymphoid cells. Immunity 36(1):92-104.
41. Ramirez JM, et al. (2010) Activation of the aryl hydrocarbon receptor reveals distinct requirements for IL-22 and IL-17 production by human T helper cells. European journal of immunology 40(9):2450-2459.
42. Sher ER, et al. (1994) Steroid-resistant asthma. Cellular mechanisms contributing to inadequate response to glucocorticoid therapy. The Journal of clinical investigation 93(1):33-39.
43. Cho YJ & Lee KE (2003) Decreased glucocorticoid binding affinity to glucocorticoid receptor is important in the poor response to steroid therapy of older-aged patients with severe bronchial asthma. Allergy and asthma proceedings : the official journal of regional and state allergy societies 24 (5) :353-358.
44. Hearing SD, Norman M, Smyth C, Foy C, & Dayan CM (1999) Wide variation in lymphocyte steroid sensitivity among healthy human volunteers. The Journal of clinical endocrinology and metabolism 84(11):4149-4154.
45. Barnden MJ, Allison J, Heath WR, & Carbone FR (1998) Defective TCR expression in transgenic mice constructed using cDNA-based alpha- and beta-chain genes under the control of heterologous regulatory elements. Immunol Cell Biol 76(1):34-40.
46. Zhang M, et al. (2003) T cell tolerance to a neo-self antigen expressed by thymic epithelial cells: the soluble form is more effective than the membrane-bound form. Journal of immunology (Baltimore, Md. : 1950) 170(8):3954-3962.
47. Silver PB, Chan CC, Wiggert B, & Caspi RR (1999) The requirement for pertussis to induce EAU is strain-dependent: B10.RIII, but not B10.A mice, develop EAU and Th1 responses to IRBP without pertussis treatment. Invest Ophthalmol Vis Sci 40(12):2898-2905.
48. Paques M, et al. (2007) Panretinal, high-resolution color photography of the mouse fundus. Invest Ophthalmol Vis Sci 48(6):2769-2774.
49. Copland DA, et al. (2008) The clinical time-course of experimental autoimmune uveoretinitis using topical endoscopic fundal imaging with histologic and cellular infiltrate correlation. Invest Ophthalmol Vis Sci 49(12):5458-5465.
50. Shi G, et al. (2008) Phenotype switching by inflammation-inducing polarized Th17 cells, but not by Th1 cells. Journal of immunology (Baltimore, Md. : 1950) 181(10) :7205-7213.
51. Howe, L.J., et al., The efficacy of systemic corticosteroids in sight-threatening retinal vasculitis. Eye, 1994. 8 (Pt 4): p. 443-7.
52. Lee, R.W.J., et al., Steroid Sensitivity in Uveitis, in Uveitis and Immunological Disorders, Progress III, U. Pleyer, Editor. 2008, Springer: Berlin.
53. Adcock, I.M., et al., Steroid resistance in asthma: mechanisms and treatment options. Curr Allergy Asthma Rep, 2008. 8(2): p. 171-8.
54. Farrell, R.J. and D. Kelleher, Glucocorticoid resistance in inflammatory bowel disease. J Endocrinol, 2003. 178(3): p. 339-46.
55. Kirwan, J.R., Glucocorticoid resistance in patients with rheumatoid arthritis. Scand J Rheumatol, 2007. 36(3): p. 165-6.
56. Hodson, E.M., N.S. Willis, and J.C. Craig, Interventions for idiopathic steroid-resistant nephrotic syndrome in children. Cochrane Database Syst Rev, 2010(11): p. CD003594.
57. Jabs, D.A., et al., Guidelines for the use of immunosuppressive drugs in patients with ocular inflammatory disorders: recommendations of an expert panel. Am J Ophthalmol, 2000. 130(4): p. 492-513.
58. Okada, A.A., Immunomodulatory therapy for ocular inflammatory disease: a basic manual and review of the literature. Ocul Immunol Inflamm, 2005. 13(5): p. 335-51.
59. Miossec, P et al (2009) NEJM ; 361(9):888-898
60. Maddur MS et al (2012) Am J Pathol; 181(1)8-18
61. Fujino S et al (2003); Gut 52(1):65-70.
62. Sakai A et al (2008) J. Immunol; 181:2898-2906
63. Li Y et al (2011); J Neuroimmunology 234(1-2): 155-160.
64. Nanzer, AM et al (2013). J Allergy Clin Immunol, 132(2):297-304
65. Heidt, S., et al. (2010). "Calcineurin inhibitors affect B cell antibody responses indirectly by interfering with T cell help." Clin Exp Immunol 159(2): 199-207.

## Claims

1. A conjugate comprising (i) an anti-CCR6 antibody and (ii) a calcineurin inhibitor for use in a method for the treatment of an inflammatory disease.

2. The conjugate for use according to claim 1, wherein the calcineurin inhibitor is tacrolimus, cyclosporin A (CsA) or voclosporin, or a therapeutically active fragment, derivative or mimetic thereof and/or, wherein the conjugate comprises an anti-CCR6 antibody linked to tacrolimus or CsA, or a therapeutically active fragment, derivative or mimetic thereof.

3. The conjugate for use according to claim 1 or 2, wherein the inflammatory disease is steroid-resistant, and/or the inflammatory disease is an autoimmune disease, and/or, wherein the inflammatory disease is a T-cell mediated inflammatory disease.

4. The conjugate for use according to any one of the preceding claims, wherein the inflammatory disease is uveitis, Behçet's disease, Vogt-Koyanagi-Harada (VKH) disease, multiple sclerosis, neuromyelitis optica, rheumatoid arthritis, psoriasis, inflammatory bowel disease (including Crohn's disease and ulcerative colitis), systemic lupus erythematosus, seronegative spondyloarthropies, Sjogren's syndrome or severe asthma, or the T-cell mediated inflammatory disease is multiple sclerosis, rheumatoid arthritis, inflammatory bowel disease, psoriasis and asthma.

5. The conjugate for use according to any one of the preceding claims, wherein in the method the conjugate is administered to a subject post-organ transplantation.

6. The conjugate for use according to any one of the preceding claims, wherein the method comprises the step of determining expression of IL-17 and/or CCR6 in a sample of T cells obtained from the subject prior to treatment, such that a subject is selected for treatment with the conjugate if the sample of T cells obtained from said subject expresses IL-17 and/or CCR6 at a higher level than a reference sample of T cells or at a higher level than a reference value of IL-17 and/or CCR6.

7. A method for identifying a subject likely to be resistant to steroid treatment, wherein the method comprises the step of determining expression of CCR6 in a sample of T cells obtained from the subject, such that expression of CCR6 in the sample at a higher level than a reference sample of T cells or at a higher level than a reference value of CCR6 indicates that the subject is likely to be resistant to steroid treatment.

8. The method according to claim 7, wherein the subject has been diagnosed with an inflammatory disease, optionally wherein the inflammatory disease is a T-cell mediated inflammatory disease. and/or, wherein the inflammatory disease is an autoimmune disease.

9. The method according to claim 8, wherein the inflammatory disease is uveitis, Behçet's disease, Vogt-Koyanagi-Harada (VKH) disease, multiple sclerosis, neuromyelitis optica, rheumatoid arthritis, psoriasis, inflammatory bowel disease (including Crohn's disease and ulcerative colitis), systemic lupus erythematosus, seronegative spondyloarthropies, Sjogren's syndrome or severe asthma, or wherein the T-cell mediated inflammatory disease is multiple sclerosis, rheumatoid arthritis, inflammatory bowel disease, psoriasis and asthma.

10. The method according to claim 7, wherein the subject is post-organ transplantation, such that the subject is likely to be at risk of steroid resistant allograft rejection.

11. A method for identifying a subject likely to benefit from treatment with a calcineurin inhibitor, wherein the method comprises the step of determining expression of CCR6 in a sample of T cells obtained from the subject, such that expression of CCR6 in the sample at a higher level than a reference sample of T cells or at a higher level than a reference value of CCR6 indicates that the subject is likely to benefit from treatment with the calcineurin inhibitor, wherein the calcineurin inhibitor is for use as a conjugate comprising (i) an anti-CCR6 antibody and (ii) a calcineurin inhibitor.

12. The method according to claim 11, wherein the calcineurin inhibitor is tacrolimus, cyclosporin A (CsA) or voclosporin, or a therapeutically active fragment, derivative or mimetic thereof.

13. The method according to claim 11 or 12, wherein the subject has previously been diagnosed with an inflammatory disease, optionally, wherein the inflammatory disease is a T-cell mediated inflammatory disease.

14. The method according to claim 13, wherein the inflammatory disease is steroid-resistant and/or, wherein the inflammatory disease is an autoimmune disease.

15. The method according to claims 13 or 14, wherein the inflammatory disease is uveitis, Behçet's disease, Vogt-Koyanagi-Harada (VKH) disease, multiple sclerosis, neuromyelitis optica, rheumatoid arthritis, psoriasis, inflammatory bowel disease (including Crohn's disease and ulcerative colitis), systemic lupus erythematosus, seronegative spondyloarthropies, Sjogren's syndrome or severe asthma, or wherein the T-cell mediated inflammatory disease is multiple sclerosis, rheumatoid arthritis, inflammatory bowel disease, psoriasis and asthma.

16. The method according to claim 11 or 12, wherein the subject is post-organ transplantation.

17. The conjugate for use according to any one of claims 1 to 6, or the method according to any one of claims 11 to 16, wherein the antibody is a Fab.

## Patentansprüche

1. Konjugat, das (i) einen Anti-CCR6-Antikörper und (ii) einen Calcineurin-Inhibitor umfasst, zur Verwendung in einem Verfahren zur Behandlung einer Entzündungserkrankung.

2. Konjugat zur Verwendung nach Anspruch 1, wobei der Calcineurin-Inhibitor Tacrolimus, Cyclosporin A (CsA) oder Voclosporin oder ein therapeutisch aktives Fragment, Derivat oder Mimetikum davon ist und/oder wobei das Konjugat einen Anti-CCR6-Antikörper an Tacrolimus oder CsA oder ein therapeutisch aktives Fragment, Derivat oder Mimetikum davon gebunden umfasst.

3. Konjugat zur Verwendung nach Anspruch 1 oder 2, wobei die Entzündungserkrankung steroidresistent ist und/oder die Entzündungserkrankung eine Autoimmunerkrankung ist und/oder die Entzündungserkrankung eine T-Zell-vermittelte Entzündungserkrankung ist.

4. Konjugat zur Verwendung nach einem der vorangegangenen Ansprüche, wobei die Entzündungserkrankung Uveitis, Morbus Behget, Vogt-Koyanagi-Harada(VKH)-Krankheit, multiple Sklerose, Neuromyelitis optica, rheumatoide Arthritis, Psoriasis, chronisch-entzündliche Darmerkrankung (einschließlich Morbus Crohn und Colitis ulcerosa), systemischer Lupus erythematodes, eine seronegative Spondyloarthropathie, Sjögren-Syndrom oder schweres Asthma ist oder die T-Zell-vermittelte Entzündungserkrankung multiple Sklerose, rheumatoide Arthritis, chronisch-entzündliche Darmerkrankung, Psoriasis oder Asthma ist.

5. Konjugat zur Verwendung nach einem der vorangegangenen Ansprüche, wobei das Konjugat in dem Verfahren einem Individuum nach einer Organtransplantation verabreicht wird.

6. Konjugat zur Verwendung nach einem der vorangegangenen Ansprüche, wobei das Verfahren den Schritt des Bestimmens der Expression von IL-17 und/oder CCR6 in einer Probe von T-Zellen umfasst, die von dem Individuum vor einer Behandlung erhalten wurde, wobei ein Individuum für eine Behandlung mit dem Konjugat ausgewählt wird, wenn die von dem Individuum erhaltene Probe von T-Zellen IL-17 und/oder CCR6 stärker exprimiert als eine Referenzprobe von T-Zellen oder stärker als ein Referenzwert von IL-17 und/oder CCR6.

7. Verfahren zur Identifizierung eines Individuums, das wahrscheinlich resistent gegen eine Steriodbehandlung ist, wobei das Verfahren den Schritt des Bestimmens der Expression von CCR6 in einer von dem Individuum erhaltenen Probe von T-Zellen umfasst, wobei eine Expression von CCR6 in der Probe stärker als in einer Referenzprobe von T-Zellen oder stärker als ein Referenzwert von CCR6 anzeigt, dass das Individuum wahrscheinlich resistent gegen eine Steroidbehandlung ist.

8. Verfahren nach Anspruch 7, wobei bei dem Individuum eine Entzündungserkrankung diagnostiziert wurde, wobei die Entzündungserkrankung gegebenenfalls eine T-Zell-vermittelte Entzündungserkrankung ist und/oder wobei die Entzündungserkrankung eine Autoimmunerkrankung ist.

9. Verfahren nach Anspruch 8, wobei die Entzündungserkrankung Uveitis, Morbus Behçet, Vogt-Koyanagi-Harada(VKH)-Krankheit, multiple Sklerose, Neuromyelitis optica, rheumatoide Arthritis, Psoriasis, chronisch-entzündliche Darmerkrankung (einschließlich Morbus Crohn und Colitis ulcerosa), systemischer Lupus erythematodes, eine seronegative Spondyloarthropathie, Sjögren-Syndrom oder schweres Asthma ist oder wobei die T-Zell-vermittelte Entzündungserkrankung multiple Sklerose, rheumatoide Arthritis, chronisch-entzündliche Darmerkrankung, Psoriasis oder Asthma ist.

10. Verfahren nach Anspruch 7, wobei das Individuum zuvor einer Organtransplantation unterzogen wurde, sodass bei dem Individuum das Risiko einer steroidresistenten Allotransplantatabstoßung besteht.

11. Verfahren zur Identifizierung eines Individuums, das wahrscheinlich von einer Behandlung mit einem Calcineurin-Inhibitor profitiert, wobei das Verfahren den Schritt des Bestimmens der Expression von CCR6 in einer vom Individuum erhaltenen Probe von T-Zellen umfasst, wobei eine Expression von CCR6 in der Probe stärker als in einer Referenzprobe von T-Zellen oder stärker als ein Referenzwert von CCR6 anzeigt, dass das Individuum wahrscheinlich von einer Behandlung mit dem Calcineurin-Inhibitor profitiert, wobei der Calcineurin-Inhibitor zur Verwendung als Konjugat dient, das (i) einen Anti-CCR6-Antikörper und (ii) einen Calcineurin-Inhibitor umfasst.

12. Verfahren nach Anspruch 11, wobei der Calcineurin-Inhibitor Tacrolimus, Cyclosporin A (CsA) oder Voclosporin oder ein therapeutisch aktives Fragment, Derivat oder Mimetikum davon ist.

13. Verfahren nach Anspruch 11 oder 12, wobei bei dem Individuum zuvor eine Entzündungserkrankung diagnostiziert wurde, wobei die Entzündungserkrankung gegebenenfalls eine T-Zell-vermittelte Entzündungserkrankung ist.

14. Verfahren nach Anspruch 13, wobei die Entzündungserkrankung steroidresistent ist und/oder die Entzündungserkrankung eine Autoimmunerkrankung ist.

15. Verfahren nach Anspruch 13 oder 14, wobei die Entzündungserkrankung Uveitis, Morbus Behget, Vogt-Koyanagi-Harada(VKH)-Krankheit, multiple Sklerose, Neuromyelitis optica, rheumatoide Arthritis, Psoriasis, chronisch-entzündliche Darmerkrankung (einschließlich Morbus Crohn und Colitis ulcerosa), systemischer Lupus erythematodes, eine seronegative Spondyloarthropathie, Sjögren-Syndrom oder schweres Asthma ist oder wobei die T-Zell-vermittelte Entzündungserkrankung multiple Sklerose, rheumatoide Arthritis, chronisch-entzündliche Darmerkrankung, Psoriasis oder Asthma ist.

16. Verfahren nach Anspruch 11 oder 12, wobei das Individuum zuvor einer Organtransplantation unterzogen wurde.

17. Konjugat zur Verwendung nach einem der Ansprüche 1 bis 6 oder Verfahren nach einem der Ansprüche 11 bis 16, wobei der Antikörper ein Fab ist.

## Revendications

1. Conjugué comprenant (i) un anticorps anti-CCR6 et (ii) un inhibiteur de la calcineurine destiné à être utilisé dans un procédé de traitement d'une maladie inflammatoire.

2. Conjugué destiné à être utilisé selon la revendication 1, dans lequel l'inhibiteur de la calcineurine est le tacrolimus, la cyclosporine A (CsA) ou la voclosporine, ou un fragment, dérivé ou mimétique thérapeutiquement actif de celui-ci/celle-ci, et/ou dans lequel le conjugué comprend un anticorps anti-CCR6 lié au tacrolimus ou à la CsA, ou à un fragment, dérivé ou mimétique thérapeutiquement actif de celui-ci/celle-ci.

3. Conjugué destiné à être utilisé selon la revendication 1 ou 2, dans lequel la maladie inflammatoire est résistante aux stéroïdes, et/ou la maladie inflammatoire est une maladie auto-immune, et/ou dans lequel la maladie inflammatoire est une maladie inflammatoire médiée par les cellules T.

4. Conjugué destiné à être utilisé selon l'une quelconque des revendications précédentes, dans lequel la maladie inflammatoire est l'uvéite, la maladie de Behçet, la maladie de Vogt-Koyanagi-Harada (VKH), la sclérose en plaques, la neuromyélite optique, la polyarthrite rhumatoïde, le psoriasis, une maladie intestinale inflammatoire (incluant la maladie de Crohn et la colite ulcéreuse), le lupus érythémateux systémique, des spondylarthropathies séronégatives, le syndrome de Sjögren ou l'asthme sévère, ou la maladie inflammatoire médiée par les cellules T est la sclérose en plaques, la polyarthrite rhumatoïde, une maladie intestinale inflammatoire, le psoriasis et l'asthme.

5. Conjugué destiné à être utilisé selon l'une quelconque des revendications précédentes, où dans le procédé, le conjugué est administré à un sujet après une transplantation d'organe.

6. Conjugué destiné à être utilisé selon l'une quelconque des revendications précédentes, où le procédé comprend l'étape consistant à déterminer l'expression de l'IL-17 et/ou de CCR6 dans un échantillon de cellules T obtenu chez le sujet avant un traitement, de sorte qu'un sujet soit sélectionné pour un traitement par le conjugué si l'échantillon de cellules T obtenu chez ledit sujet exprime l'IL-17 et/ou CCR6 à un taux plus élevé qu'un échantillon de cellules T de référence ou à un taux plus élevé qu'une valeur d'IL-17 et/ou CCR6 de référence.

7. Procédé pour identifier un sujet susceptible d'être résistant à un traitement stéroïdien, où le procédé comprend l'étape consistant à déterminer l'expression de CCR6 dans un échantillon de cellules T obtenu chez le sujet, de sorte que l'expression de CCR6 dans l'échantillon à un taux plus élevé qu'un échantillon de cellules T de référence ou à un taux plus élevé qu'une valeur de CCR6 de référence indique que le sujet est susceptible d'être résistant à un traitement stéroïdien.

8. Procédé selon la revendication 7, dans lequel le sujet a été diagnostiqué comme souffrant d'une maladie inflammatoire, facultativement dans lequel la maladie inflammatoire est une maladie inflammatoire médiée par les cellules T, et/ou dans lequel la maladie inflammatoire est une maladie auto-immune.

9. Procédé selon la revendication 8, dans lequel la maladie inflammatoire est l'uvéite, la maladie de Behçet, la maladie de Vogt-Koyanagi-Harada (VKH), la sclérose en plaques, la neuromyélite optique, la polyarthrite rhumatoïde, le psoriasis, une maladie intestinale inflammatoire (incluant la maladie de Crohn et la colite ulcéreuse), le lupus érythémateux systémique, des spondylarthropathies séronégatives, le syndrome de Sjögren ou l'asthme sévère, ou dans lequel la maladie inflammatoire médiée par les cellules T est la sclérose en plaques, la polyarthrite rhumatoïde, une maladie intestinale inflammatoire, le psoriasis et l'asthme.

10. Procédé selon la revendication 7, dans lequel le sujet a reçu une transplantation d'organe, si bien que le sujet est susceptible d'être exposé à un risque de rejet d'allogreffe résistant aux stéroïdes.

11. Procédé pour identifier un sujet susceptible de bénéficier d'un traitement par un inhibiteur de la calcineurine, où le procédé comprend l'étape consistant à déterminer l'expression de CCR6 dans un échantillon de cellules T obtenu chez le sujet, de sorte que l'expression de CCR6 dans l'échantillon à un taux plus élevé qu'un échantillon de cellules T de référence ou à un taux plus élevé qu'une valeur de CCR6 de référence indique que le sujet est susceptible de bénéficier d'un traitement par l'inhibiteur de la calcineurine, où l'inhibiteur de la calcineurine est destiné à être utilisé sous la forme d'un conjugué comprenant (i) un anticorps anti-CCR6 et (ii) un inhibiteur de la calcineurine.

12. Procédé selon la revendication 11, dans lequel l'inhibiteur de la calcineurine est le tacrolimus, la cyclosporine A (CsA) ou la voclosporine, ou un fragment, dérivé ou mimétique thérapeutiquement actif de celui-ci/celle-ci.

13. Procédé selon la revendication 11 ou 12, dans lequel le sujet a préalablement été diagnostiqué comme souffrant d'une maladie inflammatoire, facultativement dans lequel la maladie inflammatoire est une maladie inflammatoire médiée par les cellules T.

14. Procédé selon la revendication 13, dans lequel la maladie inflammatoire est résistante aux stéroïdes et/ou dans lequel la maladie inflammatoire est une maladie auto-immune.

15. Procédé selon la revendication 13 ou 14, dans lequel la maladie inflammatoire est l'uvéite, la maladie de Behçet, la maladie de Vogt-Koyanagi-Harada (VKH), la sclérose en plaques, la neuromyélite optique, la polyarthrite rhumatoïde, le psoriasis, une maladie intestinale inflammatoire (incluant la maladie de Crohn et la colite ulcéreuse), le lupus érythémateux systémique, des spondylarthropathies séronégatives, le syndrome de Sjögren ou l'asthme sévère, ou dans lequel la maladie inflammatoire médiée par les cellules T est la sclérose en plaques, la polyarthrite rhumatoïde, une maladie intestinale inflammatoire, le psoriasis et l'asthme.

16. Procédé selon la revendication 11 ou 12, dans lequel le sujet a reçu une transplantation d'organe.

17. Conjugué destiné à être utilisé selon l'une quelconque des revendications 1 à 6, ou procédé selon l'une quelconque des revendications 11 à 16, dans lequel l'anticorps est un Fab.
